# DEMANDE DE BREVET EUROPEEN

(11) **EP 3 327 128 A1**
(43) Date de publication de la demande: **30.05.2018**
(21) Numéro de dépôt: 16200144.0
(22) Date de dépôt: 23.11.2016
(51) Int. Cl.: C12N 15/70, C12N 15/74, G01N 33/94, C12Q 1/18

(54) **OUTIL GÉNÉTIQUE POUR L'ETUDE DE L'ACTIVITÉ TRANS-TRADUCTIONNELLE ET SON UTILISATION DANS LE DÉVELOPPEMENT DE NOUVEAUX ANTIBIOTIQUES**

(71) Demandeur: Université de Rennes 1, 35000 Rennes (FR); CNRS Centre National de la Recherche Scientifique, 75016 Paris (FR)
(72) Inventeur: GILLET, Reynald, 35160 Le Verger (FR); GIUDICE, Emmanuel, 35700 Rennes (FR); MACE, Kevin, 35700 Rennes (FR); DEMAY, Fanny, 35000 Rennes (FR); BLANCO, Carlos, 35250 Chasne sur Illet (FR)
(74) Mandataire: Cabinet Plasseraud

(57) **Abrégé**

La présente invention concerne des constructions génétiques de type rapporteur contenant deux gènes rapporteurs en opéron, dont l'un des gènes est lié à une étiquette peptidique reconnue par les protéases spécifiques de la *trans*-traduction. L'invention concerne également l'utilisation de ces constructions génétiques, de plasmides comprenant une construction génétique, ou de cellules bactériennes hôtes contenant une telle construction ou un tel plasmide pour étudier l'activité *trans*-traductionnelle *in vivo* ou pour identifier des composés capables d'inhiber la *trans*-traduction et donc susceptibles de présenter un intérêt en tant qu'antibiotiques.

## Description

### Contexte de l'Invention

Les antibiotiques sont des molécules permettant d'attaquer spécifiquement les bactéries, en inhibant leur croissance (action bactériostatique) ou en les détruisant (action bactéricide). L'apparition des antibiotiques dans l'arsenal thérapeutique a débuté durant la seconde guerre mondiale, peu après la découverte et la fabrication de la pénicilline. Cette découverte, qui est considérée comme l'un des progrès majeurs du XX^{ème} siècle, mena rapidement au développement de nouveaux médicaments pour le traitement de nombreuses maladies bactériennes mortelles comme la tuberculose, la pneumonie, la syphilis et le tétanos, faisant progresser l'espérance de vie de plus de dix ans.

Cependant, l'usage intensif des antibiotiques a introduit une pression de sélection aboutissant au développement inquiétant de populations de microorganismes antibiorésistants et à une baisse générale de l'efficacité thérapeutique. On sait depuis longtemps que la résistance aux antibiotiques est un phénomène naturel - les antibiotiques naturels ainsi que la résistance à ces antibiotiques datent de plus de 40 millions d'années (D'Costa et al., Nature, 2011, 477: 457-461) - mais la mauvaise conduite des traitements antibactériens, tant en médecine humaine qu'en médecine vétérinaire, a grandement accéléré ce phénomène. Ponctuelles au départ, ces résistances sont devenues massives et préoccupantes. Certaines souches bactériennes sont devenues multi-résistantes, c'est-à-dire résistantes à plusieurs antibiotiques, et d'autres toto-résistantes, c'est-à-dire résistantes à tous les antibiotiques disponibles. Ce dernier cas est heureusement encore rare, mais le phénomène est en augmentation. Il place les médecins dans une impasse thérapeutique.

Selon l'Organisation mondiale de la Santé (OMS), la résistance aux antibiotiques constitue aujourd'hui "l'une des plus graves menaces pesant sur la santé mondiale". De fait, de nombreuses infections, comme la pneumonie, la tuberculose et la gonorrhée, sont devenues plus difficiles à traiter face à la perte d'efficacité des antibiotiques utilisés pour les combattre. La résistance aux antibiotiques est à l'origine d'hospitalisations prolongées et entraîne une augmentation des dépenses médicales et de la mortalité. Dans la seule Union Européenne, on estime que les bactéries pharmacorésistantes sont responsables chaque année de 25 000 décès, avec des coûts s'élevant à plus de 1,5 milliard de dollars (US $) en frais de santé et pertes de productivité (www.who.int/mediacentre/factsheets/antibiotic-resistance/fr/# - novembre 2015). L'OMS parle d'une "ère post-antibiotique, dans laquelle les infections courantes et les blessures mineures, qui ont pu être traitées pendant des décennies, peuvent à nouveau tuer". Les plans d'action se multiplient, tant au niveau international qu'au sein des pays, pour résoudre ce problème dans le but de préserver le plus longtemps possible l'efficacité des antibiotiques disponibles (par exemple en informant et éduquant les professionnels de santé et la population, en réduisant la consommation d'antibiotiques et en optimisant l'usage de ces médicaments). Une autre piste est de redynamiser le développement de nouveaux antibiotiques, en particulier contre les formes les plus dangereuses de bactéries résistantes. Comme le souligne l'OMS, en 30 ans, seules deux nouvelles familles d'antibiotiques ont vu le jour, alors qu'il est indispensable de disposer de nouvelles molécules antibiotiques avec des mécanismes d'action originaux.

Le mécanisme du contrôle-qualité de la synthèse protéique appelé *trans*-traduction, qui fait l'objet d'un des programmes de recherche de l'équipe des présents Inventeurs, est particulièrement attrayant pour le développement de nouveaux antibiotiques à large spectre. En effet, la traduction du code génétique en protéines *via* le ribosome est le fondement de la vie pour toutes les cellules. Au vu de la quantité de données biologiques à traiter, il arrive régulièrement que le ribosome se grippe et mette ainsi en péril la survie de la cellule. Chez les bactéries, le principal mécanisme de sauvetage des ribosomes grippés est la *trans*-traduction, portée par un acide ribonucléique (ARN) hybride: l'ARN transfert-messager (ARNtm). Etonnamment, ce système est essentiel à la survie de nombreuses bactéries pathogènes (*Staphylococcus aureus, Mycobaterium tuberculosis, Neisseria gonorrhoeae, Helicobacter pylori,* et *Shigella flexneri*) et elle est requise pour la virulence d'autres espèces (*Salmonella, Yersinia,* et *Francisella*). Les connaissances structurales et biologiques acquises lors de la dernière décennie ont permis d'identifier la *trans*-traduction comme une cible antibiotique particulièrement attrayante puisqu'elle est absente chez les cellules eucaryotes (Himeno et al., Front Genet., 2014, 5: 66, doi: 10.3389/fgene.2014.00066; Giudice et al., Front Microbiol., 2014, 5: 113, doi: 10.3389/fmicb.2014.00113). La découverte récente du mode d'action de la pyrazinamide (PIRILENE^{®}), le plus ancien antituberculeux utilisé en clinique, sur la *trans*-traduction a amené la preuve de concept manquante (Shi et al., Science, 2011, 333 : 1630-1632), confirmant l'ARNtm et ses partenaires, en particulier la petite protéine B (SmpB - Small Protein B), comme des cibles attrayantes d'une nouvelle classe de molécules antibiotiques à large spectre.

Malgré certains progrès récents (Ramadoss et al., P.N.A.S USA, 2016, 110: 10282-10287), un frein au développement de telles molécules anbiotiques est le manque de procédés simples et rapides pour évaluer les effets de composés tests sur la *trans-*traduction. Il existe donc toujours dans l'art, un besoin de disposer de nouveaux outils pour étudier l'activité *trans*-traductionnelle chez différentes bactéries.

### Résumé de l'Invention

D'une façon générale, l'invention permet de détecter spécifiquement l'activité *trans*-traductionnelle *in vivo,* dans différentes conditions de culture, à l'aide d'un système rapporteur rapide, fiable et économique. Plus spécifiquement, comme indiqué plus haut, la *trans*-traduction, assurée par l'ARNtm et la protéine SmpB, est un mécanisme de sauvetage des ribosomes bloqués lors de la traduction bactérienne. Au cours de ce processus, l'ARNtm ajoute une étiquette peptidique (ou tag) sur la protéine incomplète bloquée dans le ribosome, ce qui permet la dégradation rapide de la protéine taguée par les protéases. Afin de mesurer l'activité *trans*-traductionnelle, le système rapporteur de la présente invention utilise cette capacité d'ajout de l'étiquette peptidique et comprend également un témoin de la voie de dégradation par les protéases de façon à assurer la spécificité du rapporteur. Ainsi, alors que les méthodes déjà existantes sont sans contrôle interne et peu reproductibles car elles nécessitent l'utilisation de plusieurs cultures avec différentes constructions plasmidiques pour vérifier la spécificité du système rapporteur, l'outil génétique de la présente invention comporte à la fois un rapporteur de la *trans-*traduction (l'étiquette peptidique) et un contrôle interne (témoin de la voie de dégradation par les protéases), tous deux étant placés sous le contrôle du même promoteur (voir les Exemples ci-dessous). La présence des deux rapporteurs dans la même construction génétique permet d'augmenter la fiabilité et la reproductibilité et de limiter les coûts d'expérimentation. De plus, contrairement aux méthodes déjà existantes qui ne sont applicables qu'à *Escherichia coli,* l'outil génétique selon l'invention est tel qu'il est facilement transposable chez différentes espèces et souches bactériennes. Enfin, le système rapporteur selon l'invention est adapté à une utilisation dans différentes conditions de culture (biofilm, gélose ou cultures liquides) et permet également le suivi dynamique de la *trans*-traduction avec un suivi des bactéries en phase exponentielle comme en phase stationnaire. La visualisation du résultat peut être effectuée par une grande variété de techniques de mesure de fluorescence (microscopie de fluorescence, fluorimétrie, etc...). Le système a également été optimisé pour la lecture sur plaque multi-puits de façon à faciliter le criblage de composés actifs. En effet, un système rapporteur selon l'invention trouve application non seulement comme un outil indispensable à la recherche fondamentale sur la *trans*-traduction mais également dans le criblage de composés en vue d'identifier de nouveaux antibiotiques à large spectre.

En conséquence, dans un premier aspect, la présente invention concerne une construction génétique destinée à être utilisée dans une espèce ou souche de bactérie, ladite construction génétique comprenant:
- la séquence d'un premier gène rapporteur,
- la séquence d'un deuxième gène rapporteur, et
- la séquence d'un promoteur,
   et la construction génétique étant caractérisée en ce que:
   - les premièr et deuxième gènes rapporteurs sont différents et se trouvent sous le contrôle du promoteur,
   - la séquence du premier gène rapporteur est liée de façon opérationnelle:
      - en 5': à un site d'initiation de traduction, et
      - en 3': à une séquence codant pour une étiquette peptidique et terminée par un codon stop, l'étiquette peptidique étant dégradée par les protéases spécifiques de la *trans*-traduction de l'espèce ou souche de bactérie dans laquelle la construction génétique est destinée à être utilisée,
   - la séquence du deuxième gène rapporteur est liée de façon opérationnelle:
      - en 5': à un site d'initiation de traduction, et
      - en 3': à la séquence d'un terminateur fort, et
   - le site d'initiation de traduction en 5' de la séquence du premier gène rapporteur et le site d'initiation de traduction en 5' de la séquence du deuxième gène rapporteur sont identiques.

Dans certains modes de réalisation, les premier et deuxième gènes rapporteurs sont des gènes rapporteurs codant pour des protéines ayant une activité quantifiable. Par exemple, les protéines ayant une activité quantifiable peuvent être des protéines fluorescentes.

Dans certains modes de réalisation de l'invention, le site d'initiation de traduction comprend une séquence correspondant à un site de fixation au ribosome.

Dans certains modes de réalisation, le terminateur fort est une succession de codons rares, une séquence de reconnaissance par des ribonucléases, une séquence de blocage des ribosomes, ou un terminateur comprenant au moins une structure en tige-boucle.

Dans certains modes de réalisation, la construction génétique comprend en outre des sites de restriction permettantà la construction génétique d'être insérée dans un plasmide et des sites de restrictions permettant la modification ou le remplacement d'au moins un élément choisi parmi le promoteur, le premier gène rapporteur, le deuxième gène rapporteur, l'étiquette peptidique, et le terminateur fort. Ces sites de restriction peuvent, par exemple, être reconnus par des enzymes choisis parmi BamHI, HindIII, KpnI, NdeI, ApaI, XbaI, BglII, et EcoRI.

Dans certains modes de réalisation, la construction génétique est destinée à être utilisée dans *E. coli*; est transposable à d'autres bactéries Gram-; sa séquence est la séquence SEQ ID NO: 26.

La présente invention concerne également un vecteur ou un plasmide comprenant une construction génétique telle que divulguée ici.

Dans certains modes de réalisation préférés, le vecteur ou plasmide comprend en outre un promoteur inductible, par exemple un promoteur inductible par un inducteur chimique, tel que l'IPTG.

L'invention concerne également une cellule bactérienne hôte comprenant une construction génétique telle que décrite ici, ou un vecteur ou plasmide comprenant une telle construction génétique.

L'invention concerne aussi l'utilisation d'une construction génétique, ou d'un vecteur ou plasmide ou encore d'une cellule bactérienne hôte selon l'invention, pour étudier la *trans*-traduction et/ou l'activité protéasique en lien avec la *trans*-traduction.

L'invention concerne en outre un procédé de criblage de composés capables d'inhiber la *trans*-traduction bactérienne, comprenant des étapes consistant à:
- incuber une cellule bactérienne transformée par un vecteur ou plasmide comprenant une constrution génétique décrite ici, avec un composé test, et
- détecter la présence ou l'absence, ou déterminer la quantité, de la protéine codée par le premier gène rapporteur et détecter la présence ou l'absence, ou déterminer la quantité, de la protéine codée par le deuxième gène rapporteur.

Dans certains modes de réalisation, le premier gène rapporteur code pour une première protéine fluorescente et le deuxième gène rapporteur code pour une deuxième protéine fluroescente, et l'étape de détection comprend mesurer la fluorescence émise par première protéine fluorescente et mesurer la fluorescence émise par la deuxième protéine fluroescente.

La présente invention concerne enfin un kit comprenant une construction génétique telle que décrite ici, ou un vecteur ou plasmide comprenant une telle construction génétique, ou encore une cellule bactérienne hôte comprenant un tel vecteur ou plasmide.

Une description plus détaillée de certains modes de réalisation préférés de l'invention est donnée ci-dessous.

### Légendes des Figures

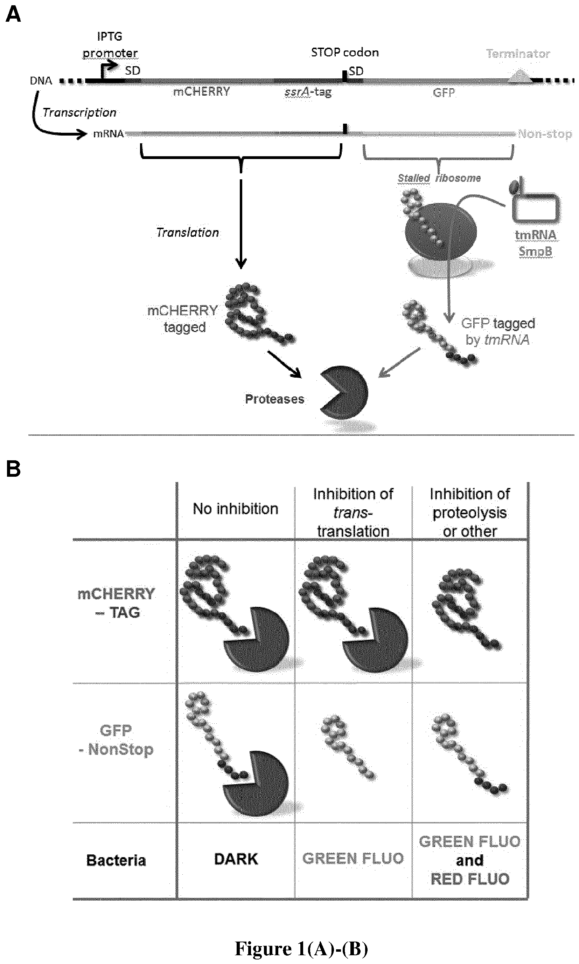

**Figure 1****:** Schéma explicatif du système rapporteur de la *trans*-traduction (**A**) et les différents résultats possibles en fonction de la situation (**B**).

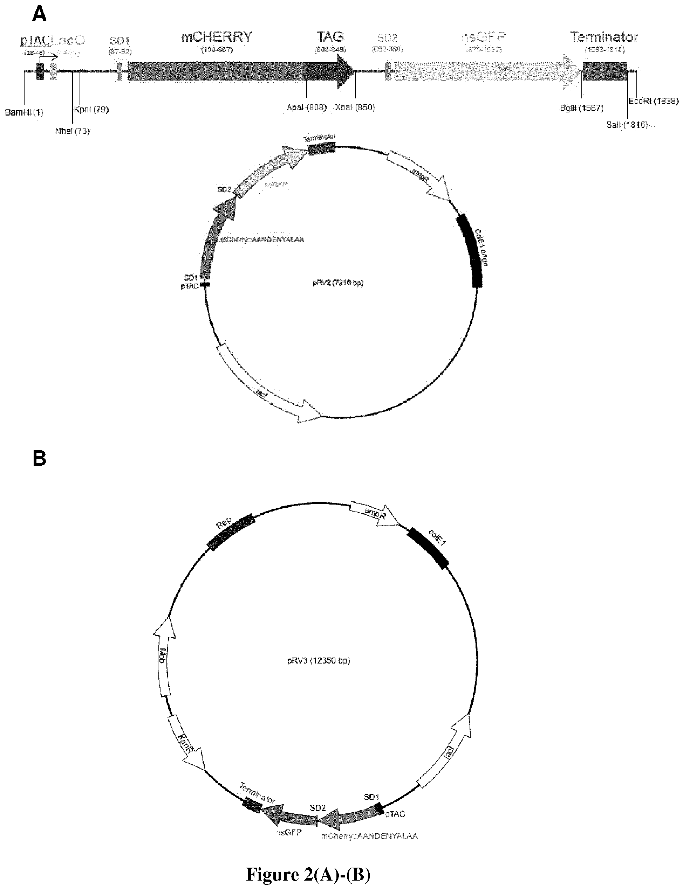

**Figure 2****: (A)** Carte plasmidique du vecteur rapporteur chez *E. coli* (pRV2) avec indication des principaux éléments du vecteur. Système inductible par IPTG (LacI - gène codant pour l'opérateur qui régule la transcription des gènes rapporteurs - LacO: site de fixation de l'opérateur) et démarrage de la transcription *via* le promoteur ptac (région -10, -35). SD1 et SD2 = Shine delgarno, sites de fixation des ribosomes (un SD par protéine, SD identique). mCHERRY (rouge) + tag *E.coli* (violet). La GFP (**vert**) avec son terminateur (vert foncé). Plasmide Ampicilline résistant (AmpR) à grand nombre de copie par bactérie (Origine ColE1). . Des sites de restrictions uniques et présents dans d'autres espèces bactériennes sont présents de part et d'autre de la construction et du tag mCHERRY. **(B)** Carte plasmidique du vecteur rapporteur à large spectre de réplication chez les bactéries à Gram négatif (pRV3) avec indication des principaux éléments du vecteur. Kan/NéoR: gène de résistance à la kanamycine et néomycine. Rep: origine de réplication du plasmide pBBR1-MCS2. Mob: protéine de mobilisation permettant le transfert du plasmide par conjugaison. LacI: gène codant pour le répresseur qui régule la transcription des gènes rapporteurs. LacO: site de fixation de LacI. Région -35 -10: promoteur pour la transcription. SD1 et SD2: Site de fixation des ribosomes, permet la traduction des gènes rapporteurs.

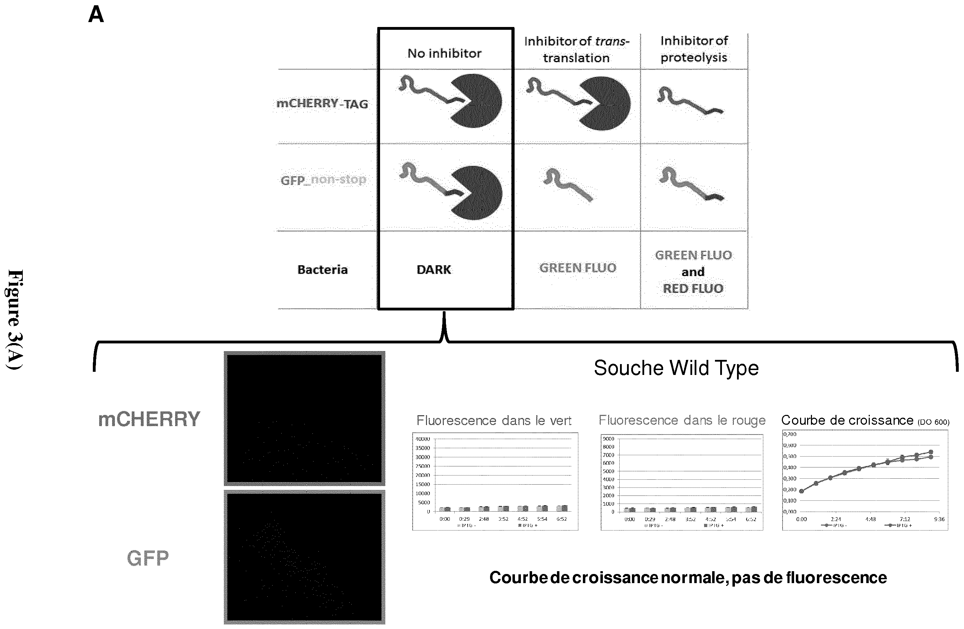

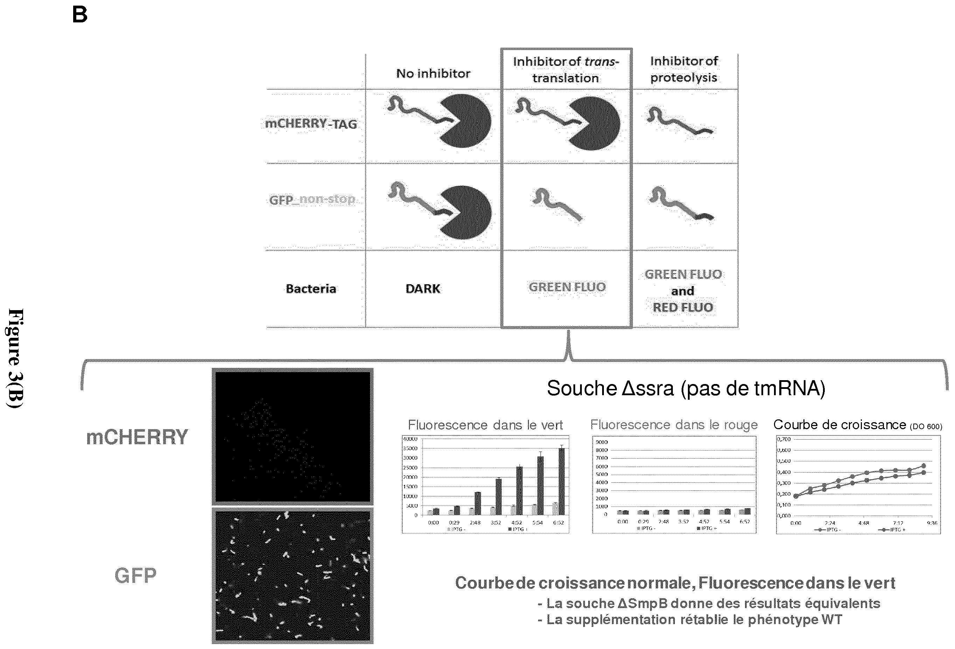

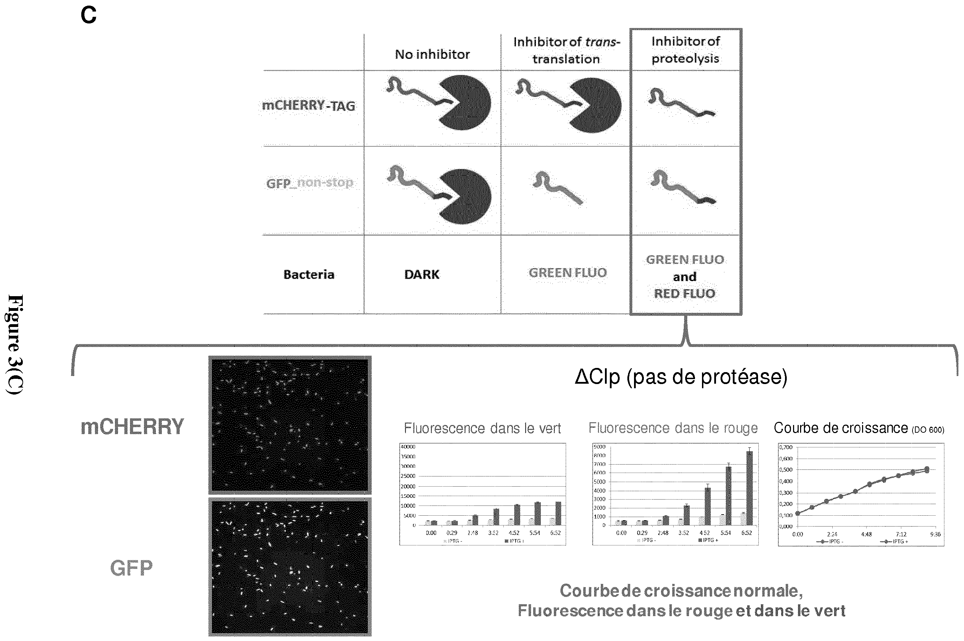

**Figure 3****: (A)** Effets attendus dans une souche sauvage: les protéases sont efficaces et la *trans*-traduction est fonctionnelle. Les deux protéines reportrices sont dégradées. Ni la fluorescence verte, ni la fluorescence rouge ne sont observées. **(B)** Effets attendus lorsque la souche sauvage se trouve en présence d'inhibiteur de la *trans*-traduction. Les protéases sont toujours efficaces donc mCherry est dégradée: la fluorescence rouge n'est pas observée. Par contre, la *trans*-traduction n'est pas fonctionelle, la GFP n'est donc pas étiquetée par l'ARNtm: la protéine s'accumule dans la bactérie sans pouvoir être dégradée, une fluorescence verte est donc observée. **(C)** Effets attendus dans une souche où les protéases sont inhibées. Dans ce cas les protéases ne sont pas efficaces, la protéine mCherry s'accumule donc dans la bactérie. La *trans*-traduction est fonctionnelle, la GFP est donc étiquetée mais il n'y a pas de protéases pour la dégrader, la protéine GFP s'accumule également dans la bactérie, les deux fluroescences, rouge et verte, sont donc observées.

### Description Détaillée de l'Invention

Comme mentionné ci-dessus, la présente invention concerne un outil génétique permettant d'étudier l'activité *trans*-traductionnelle *in vivo.* Cet outil, qui est facilement transposable à différentes espèces et souches de bactéries, trouve en particulier application dans le criblage de composés capables d'inhiber la *trans*-traduction et donc susceptibles de présenter un intérêt en tant qu'antibiotiques.

### I - Outils Génétiques pour l'Etude de l'Activité trans-Traductionnelle

Dans un premier aspect, l'invention concerne une construction génétique de type gène rapporteur, et un vecteur (plasmide) comprenant la construction génétique.

### Construction ou Cassette d'Expression de Type Gène Rapporteur

Les termes "construction de type gène rapporteur" et "cassette d'expression de type gène rapporteur" sont utilisés ici de façon interchangeable. Ils désignent une organisation de différentes séquences d'ADN qui comprend, en particulier, la séquence d'un gène rapporteur insérée en orientation sens et liée opérationnellement à un ou plusieurs éléments permettant l'expression du gène rapporteur et éventuellement sa régulation dans une cellule donnée. Le terme "gène rapporteur" désigne un gène dont le produit (une protéine) possède une caractéristique lui permettant d'être observé ou détecté. Les termes "lié opérationnellement" et "lié de façon opérationnelle" sont utilisés indifféremment et font référence à un lien fonctionnel entre les éléments permettant l'expression du gène et éventuellement sa régulation (séquences régulatrices 5' et 3') et la séquence du gène rapporteur qu'elles contrôlent. Les constructions génétiques selon l'invention ont la particularité de comprendre deux gènes rapporteurs en opéron.

Plus spécifiquement, une construction génétique selon l'invention est destinée à être utilisée dans une espèce ou souche de bactérie et comprend:
- la séquence d'un premier gène rapporteur,
- la séquence d'un deuxième gène rapporteur, et
- la séquence d'un promoteur.

La construction génétique est caractérisée en ce que:
- les premier et deuxième gènes rapporteurs sont différents et se trouvent sous le contrôle du même promoteur,
- la séquence du premier gène rapporteur est liée de façon opérationnelle:
   - en 5': à un site d'initiation de traduction, et
   - en 3': à une séquence codant pour une étiquette peptidique et terminée par un codon stop, l'étiquette peptidique étant dégradée par les protéases spécifiques de la *trans*-traduction de l'espèce ou souche de bactérie dans laquelle la construction génétique est destinée à être utilisée,
- la séquence du deuxième gène rapporteur est liée de façon opérationnelle:
   - en 5': à un site d'initiation de traduction, et
   - en 3': à la séquence d'un terminateur fort, et
- le site d'initiation de traduction en 5' de la séquence du premier gène rapporteur et le site d'initiation de traduction en 5' de la séquence du deuxième gène rapporteur sont identiques.

L'expression "destinée à être utilisée dans une espèce ou souche de bactérie", employée ici pour définir ou caractériser une construction génétique, signifie que les éléments de la construction sont sélectionnés pour permettre la transcription des deux gènes rapporteurs dans une cellule bactérienne et que les éléments qui sont spécifiques d'une espèce ou souche de bactérie sont sélectionnés pour permettre la transcription des deux gènes rapporteurs dans une cellule de l'espèce ou souche de bactérienne dans laquelle la construction génétique est destinée à être utilisée. L'homme du métier sait sélectionner de tels éléments. En particulier, l'homme du métier sait sélectionner un promoteur approprié, et une étiquette peptidique appropriée (voir plus loin).

L'espèce ou souche de bactérie dans laquelle la construction génétique est destinée à être utilisée peut être n'importe quelle espèce ou souche de bactérie. En particulier, la bactérie peut être une bactérie Gram positif (Gram+) ou bien une bactérie Gram négatif (Gram-). Une "bactérie Gram+" est une bactérie qui a une structure unimembranée et qui apparaît mauve par la technique de coloration de Gram. Les bactéries Gram+ incluent, sans limitation, les bactéries appartenant aux genres *Staphylococcus, Micrococcus, Lactococcus, Lactobacillus, Clostridium, Bacillus, Streptococcus, Corynebacterium, Enterococcus,* et *Listeria.* Une "bactérie Gram-" est une bactérie qui a, sauf exception, une structure bimembranée et qui apparaît rose par la technique de coloration de Gram. Les bactéries Gram- incluent, sans limitation, les bactéries appartenant aux genres *Bordetella, Salmonella, Enterobacter, Klebsiella, Shigella, Yersinia, Escherichia coli, Vibrio, Pseudomonas, Neisseria, Haemophilus,* et *Agrobacterium.*

### Promoteur

Par "promoteur", on entend tout polynucléotide capable de réguler l'expression, dans une cellule, d'une séquence nucléotidique à laquelle il est lié de façon opérationnelle. Dans le contexte de l'invention, un promoteur est capable d'exercer son action régulatrice dans une cellule de bactérie. Ainsi, dans le contexte de l'invention, une séquence régulatrice de type promoteur est une région régulatrice reconnue par une ARN polymérase dans une cellule bactérienne hôte et capable d'initier la transcription de la séquence du premier gène rapporteur et de la séquence du deuxième gène rapporteur dans la cellule bactérienne hôte. Les deux gènes rapporteurs sont donc sous le contrôle du même promoteur. Le fait d'utiliser un seul promoteur pour les deux gènes rapporteurs (en opéron) permet une expression transcriptionnelle identifque des deux rapporteurs.

Le promoteur peut être homologue à la cellule bactérienne hôte dans laquelle la construction génétique est destinée à être utilisée ou, alternativement, peut être hétérologue à la cellule bactérienne hôte. De plus, le promoteur peut être une séquence naturelle (c'est-à-dire une séquence existant dans la nature) ou une séquence synthétique (c'est-à-dire une séquence n'existant pas en tant que telle dans la nature - par exemple une séquence consensus pour une espèce bactérienne donnée).

Les promoteurs adaptés à la transcription dans des cellules bactériennes hôtes incluent, sans limitation, dans le cas particulier de la bactérie *E. Coli,* les promoteurs lac, lacUV5, tac, trc, trp, araBAD, phoA, recA, proU, cst-I, tetA, cadA, nar, p_{L}, cspA, SP6, T7, T3, T5, T4, nprM, VHb. Pour une autre espèce de bactérie, le promoteur est promoteur fort chez cette bactérie, par exemple un promoteur d'ARN ribosomique.

Ce promoteur est éventuellement associé à un régulateur, le choix du régulateur étant dépendant de l'espèce bactérienne. De nombreux exemples de régulateurs ont été décrits, les plus classiques étant les protéines LacI et TetR. Dans cette éventualité, un opérateur adéquat est associé au promoteur. L'homme du métier sait sélectionner le promoteur et le régulateur associés les plus adaptés en fonction de l'utilisation finale de la construction génétique rapportrice.

Des séquences consensus de promoteurs adaptés à la transcription dans des cellules bactériennes sont connues dans l'art. Par exemple, quand le système rapporteur est destiné à être utilisé dans *Escherichia coli* (*E. coli*), la séquence consensus décrite par Kanaya et Kudo (Nucleic Acids Symp., 1991, 25: 41-42).

### Gènes Rapporteurs

Comme indiqué plus haut, le terme "gène rapporteur" désigne un gène dont le produit (une protéine) possède une caractéristique lui permettant d'être observé ou quantifié, par exemple par détection d'une bioluminescence (par exemple fluorescence) ou d'une activité enzymatique. Ainsi, un gène rapporteur utile dans la mise en oeuvre de la présente invention peut être choisi parmi les gènes rapporteurs codant pour une protéine fluorescente, et les gènes rapporteurs codant pour une enzyme dont l'action provoque l'apparition d'un produit coloré ou d'un phénotype facilement caractérisable, ou toute autre protéine ou ayant toute autre activité quantifiable.

Dans certains modes de réalisation, un gène rapporteur compris dans une construction génétique selon l'invention est choisi parmi les gènes rapporteurs codant pour une enzyme, comme par exemple le gène de la luciférase de Vibrio (Lux), le gène de la bêta-galactosidase (LacZ) ou d'une hydrolase (β-glucoronidase, phosphatase-alcaline), le gène de la chloramphénical acétyltransférase (CAT), le gène de la bêta-lactamase (TEM-1) et le gène de structure d'une protéine toxique (toxique des couples toxines antitoxine, sacB, nucléase...).

Dans d'autres modes de réalisation, un gène rapporteur compris dans une construction selon l'invention est choisi parmi les gènes rapporteurs codant pour une protéine fluorescente.

Préférablement, pour une application automatisée, les deux gènes rapporteurs compris dans une construction selon l'invention sont des gènes rapporteurs codant pour une protéine fluorescente.

Plus préférablement encore, le premier gène rapporteur code pour une première protéine fluorescente, le deuxième gène rapporteur code pour une deuxième protéine fluorescente, et les première et deuxième protéines fluorescentes sont différentes. Chacune de ces protéines fluorescentes (ou sondes fluorescentes) doit être adaptée à l'exploration cellulaire. Par "protéines fluorescentes différentes", on entend des protéines qui fluorescent dans des régions différentes du spectre optique, ou plus spécifiquement des protéines qui émettent une fluorescence à des longueurs d'onde suffisamment différentes pour qu'elles puissent être distinguées en utilisant une méthode classique de détection ou de mesure de fluorescence. Ainsi, la première protéine fluorescente peut émettre dans le rouge (longueur d'onde d'émission comprise entre environ 605 nm et 780 nm) et la deuxième protéine fluorescente peut émettre dans le vert (longueur d'onde d'émission comprise entre environ 510 nm et 570 nm). Alternativement, la première protéine fluorescente peut émettre dans le bleu (longueur d'onde comprise entre environ 460 nm et 480 nm) et la deuxième protéine fluorescente peut émettre dans l'orangé (longueurs d'onde d'émission comprise entre environ 585 nm et 605 nm). L'homme du métier sait sélectionner deux protéines fluorescentes différentes pour développer un système rapporteur selon l'invention.

La protéine fluorescente la plus connue est la GFP (ou Green Fluorescent Protein), qui fluoresce dans le vert et qui est issue d'une méduse (*Aequorea victoria*). Plus d'une centaine de protéines fluorescentes, dérivées soit de la GFP soit de la RFP (Red Fluorescent Protein ou DsRed) sont clonées aujourd'hui. Ces protéines fluorescentes sont divisées en 7 catégories en fonction de leur maximum d'émission: les protéines fluorescentes émettant dans le bleu (appelées les BFPs ou Bleu Fluorescent Proteins, émettant entre 400 nm et 470 nm), dans le cyan (les CFPs ou Cyan Fluorescent Proteins, émettant entre 471 nm et 500 nm), dans le vert (les GFPs ou Green Fluorescent Proteins, émettant entre 501 nm et 520 nm), dans le jaune (les YFPs ou Yellow Fluorescent Proteins, émettant entre 521 nm et 550 nm), l'orange (les OFPs ou Orange Fluorescent Proteins, émettant entre 551 nm et 575 nm), dans le rouge (les RFPs ou Red Fluorescent Proteins, émettant entre 576 nm et 610 nm) et dans le rouge lointain (les FRFPs ou Far-Red Fluorescent Proteins, émettant entre 611 nm et 660 nm) (Newman et al., Chem. Rev., 2011, 111: 3614-3666).

Ainsi, un gène rapporteur qui peut être utilisé dans le contexte de la présente invention est un gène codant pour une protéine fluorescente appartenant à la catégorie des BFPs (comme par exemple les protéines Sirius, EBFP, SBFP2, EBFP2, Azurite, mKalama1, TagBFP et mBlueberry2), des CFPs (comme par exemple SCFP3A, mTurquoise, mseCFP, Cerulean, ECFP, CyPet, TagCFP, mTFP1, Midori-ishi Cyan, et mUKG), des GFPs (comme par exemple TurboGFP, AceGFP, Azami Green, ZsGreen, TagGFP2, EGFP, mWasabi, Emerald, Superfolder GFP, Sapphire, T-Sapphire, et SGFP2), des YFPs (comme par exemple TagYFP, mAmetrine, EYFP, Topaz, SYFP2, Venus, mCitrine, Ypet, PhiYFP, et ZsYellow1), des OFPs (comme par exemple mBanana, mOrange, mKOκ, Kusabira Orange2, mOrange2, OFP, et TurboRFP), des RFPs (comme par exemple tdTomato, DsRed2, DsRed, DsRed-Express, TagRFP, TagRFP-T, mTangerine, DsRed-Express2, DsRed-Max, AsRed2, mApple, mStrawberry, mRuby, mRFP1, tdRFP611 et mCherry), ou des FRFPs (comme par exemple eqFP611, tdKeima, mKeima, mRaspberry, tdRFP639, tdKatushka2, mKate, mKate2, mPlum, mNeptune et AQ143).

Dans certains modes de réalisation particuliers de l'invention, le premier gène rapporteur code pour la mCherry et le deuxième gène rapporteur code pour GFP.

### Etiquette Peptidique dégradée par les Protéases de la Trans-traduction

Par "étiquette peptidique dégradée par les protéases spécifiques de la *trans-*traduction", on entend un peptide ayant une séquence identique à la séquence de l'étiquette peptidique ajoutée, par l'ARNtm, à la protéine incomplète bloquée dans le ribosome, au cours du processus de *trans*-traduction dans l'espèce ou souche de bactérie dans laquelle la construction génétique est destinée à être utilisée. Cette étiquette est également appelée "étiquette SsrA", "SsrA-tail", "queue SsrA" ou "peptide de dégradation SsrA" ("degradation SsrA tag" en anglais), du nom du gène SsrA qui code l'ARNtm chez les bactéries , ou encore "séquence peptidique encodée par l'ARNtm".

La séquence de l'étiquette peptidique dégradée par les protéases spécifiques de la *trans*-traduction est donc bactérie-spécifique. Ainsi, l'étiquette peptidique est choisie en fonction de l'utilisation finale de la construction génétique, par exemple l'étude du mécanisme de *trans*-traduction chez une espèce ou souche de bactérie donnée ou l'identification de composés capables d'inhiber la *trans*-traduction chez une espèce ou souche de bactérie donnée.

Dans le cas d'*E*. *coli,* où le mécanisme de *trans*-traduction a été étudiée de façon extensive (Gottesman et al., Genes and Development, 1998, 12: 1338-1347; Song and Eck, Mol. Cell, 2003, 12: 75-86; Kirsten et al., Nature Reviews, 2009, 7: 589-599), l'étiquette peptidique dégradée par les protéases spécifiques de la *trans*-traduction a la séquence suivante:
ANDENYALAA (SEQ ID NO: 1),
   et la séquence codant pour cette étiquette peptidique est:
   GGGCCCGCAGCAAACGACGAAAACTACGCTCTCGCGGCT (SEQ ID NO: 2).

Dans les modes de réalisation de l'invention où le système rapporteur est destiné à être utilisé dans *E. coli,* la construction génétique selon l'invention peut donc comprendre la séquence (SEQ ID NO: 2) du gène codant pour l'étiquette peptidique de séquence SEQ ID NO: 1. Cette séquence est liée en 3' à la séquence du premier gène rapporteur (par exemple du premier gène codant pour la première protéine fluorescente).

La construction génétique selon l'invention peut être adaptée à d'autres espèces bactériennes (ou souches bactériennes) qu'*E*. *coli,* en choisissant des étiquettes peptidiques dégradées par les protéases spécifiques de la *trans*-traduction chez ces espèces ou souches bactériénnes. Les séquences de telles étiquettes peptidiques sont connues dans l'art (voir en particulier la liste fournie sur le site: www.ag.auburn.edu/mirror/tmRDB/peptide/peptidephylolist.html). On peut citer par exemple les séquences présentées dans le tableau 1.

Dans une cassette d'expression selon l'invention, la séquence codant pour l'étiquette peptidique dégradée par les protéases spécifiques de la *trans*-traduction est insérée en phase avant le codon stop de la séquence du premier gène rapporteur.

**Tableau 1. Séquences d'Etiquettes Peptidiques et Bactéries Associées**

| **Bactérie(s)** | **Séquence de l'Etiquette** | **SEQ ID NO :** |
|---|---|---|
| *Escherichia coli* | ANDENYALAA | SEQ ID NO: 1 |
| *Yersinia pestis* | | |
| *Vibrio cholerae* (et également V. fischeri, V. parahaemolyticus RIMD 2210633 et V. vulnificus) | | |
| *Shigella dysenteriae* (souche M131649) et *Shigella flexneri* | | |
| *Klebsiella pneumoniae* | | |
| *Serratia marcescens* | | |
| *Enterobacter* aerogenes (souche ATCC 13048) | | |
| *Clostridium tetani* | ADDNFVLAA | SEQ ID NO: 3 |
| *Mycobacterium tuberculosis* | ADSHQRLAA | SEQ ID NO: 4 |
| *Mycobacterium tuberculosis souche 210* | ADSHQRDYALAA | SEQ ID NO: 5 |
| *Mycobacterium leprae* | ADSYQRDYALAA | SEQ ID NO: 6 |
| *Corynebacterium diphtheriae* | AENTQRDYALAA | SEQ ID NO: 7 |
| *Chlamydia trachomatis* | AEPKAECEIISFADLEDLRVAA | SEQ ID NO: 8 |
| *Borrelia burdorferi* | AKNNNFTSSNLVMAA | SEQ ID NO: 9 |
| *Streptococcus pneumoniae* (souche 670) | AKNNTSYALAA | SEQ ID NO: 10 |
| *Streptococcus pyogenes* | AKNTNSYALAA | SEQ ID NO: 11 |
| *Pseudomonas aeruginosa* | ANDDNYALAA | SEQ ID NO: 12 |
| *Haemophilus influenzae* | ANDEQYALAA | SEQ ID NO: 13 |
| *Bordetella pertussis* et *Bordetella parapertussis* | ANDERFALAA | SEQ ID NO: 14 |
| *Neisseria gonorrhoeae* et *Neisseria meningitides* | ANDETYALAA | SEQ ID NO: 15 |
| *Clostridium botulinum* | ANDNFALAA | SEQ ID NO: 16 |
| *Treponema pallidum* | ANSDSFDYALAA | SEQ ID NO: 17 |
| *Staphylococcus aureus* (souches COL, NCTC8325, EMRSA-16, MSSA 476, Mu50, et N315 et sbsp. aureus MW2) | GKSNNNFAVAA | SEQ ID NO: 18 |
| *Helicobacter pylori* | VNNTDYAPAYAKAA | SEQ ID NO: 19 |

### Site d'Initiation de Traduction

Dans une cassette d'expression selon l'invention, la séquence du premier gène rapporteur et la séquence du deuxième gène rapporteur sont chacune liées de façon opérationnelle, en 5', à un site d'initiation de traduction. Pour obtenir un niveau d'expression traductionnelle identique des protéines rapportrices, le site d'initiation de traduction placé en amont du premier gène rapporteur et le site d'initiation placé en amont du deuxième gène rapporteur sont identiques.

Le site d'initiation, dans une constrution génétique selon l'invention, comprend une séquence correspondant à un site de fixation au ribosome (SD ou RBS), qui permet l'initiation de la synthèse protéique (traduction). Les sites de fixation au ribosome sont complémentaires à l'extremity de l'ARN 16S, généralement riches en purines (A et G) et longs de 3 à 9 nucléotides consécutifs. De telles séquences sont connues dans l'art. Dans les modes de réalisation de l'invention où la construction génétique est destinée à être utilisée dans d'*E*. *Coli,* la séquence correspondant à un site de fixation au ribosome peut être: AAGGAGA, qui est une séquence consensus pour *E. coli* (Barrick et al., Nucleic Acids Res., 1994, 22: 1287-1295).

Une séquence correspondant à un site de fixation au ribosome est généralement placée 6 à 12 nucléotides en amont du codon de démarrage du gène. Un site d'initiation, dans une constrution génétique selon l'invention, est donc constitué d'une séquence correspondant à un site de fixation au ribosome et de 6 à 12 nucléotides supplémentaires.

Dans les modes de réalisation de l'invention où la construction génétique est destinée à être utilisée dans d'*E*. *Coli,* les 6 à 12 nucléotides supplémentaires peuvent par exemple être: TATACAT - une séquence arbitraire.

### Terminateur Fort

Dans une cassette d'expression selon l'invention, la séquence du deuxième gène rapporteur est liée de façon opérationnelle, en 3', à la séquence d'un terminateur fort. Les termes "terminateur" et "terminateur de transcription" sont utilisés ici indifféremment. Ils désignent une séquence qui marque la fin de la transcription d'un gène ou d'un opéron en ARN messager. Le terme "terminateur fort" désigne ici un terminateur qui stoppe prématurément la transcription, et conduit à la synthèse d'un ARNm (ARN messager) dépourvu de codon stop sur le dernier gène de l'opéron. Ainsi, dans la construction génétique de l'invention, la séquence du deuxième gène rapporteur ne contient pas de codon stop ou, en d'autres termes, le codon stop du deuxième gène rapporteur est remplacé par la séquence du terminateur. Le terminateur fort est donc placé à la fin de la séquence du deuxième gène pour que la transcription s'arrête à la fin de l'ARNm sans rencontrer de codon stop. Cela a pour conséquence de bloquer l'ARNm dans le ribosome et d'induire le processus de *trans*-traduction. L'absence de codon stop impose que, dans l'opéron, la séquence du second gène rapporteur soit en aval du premier gène rapporteur qui est lié à l'étiquette peptidique.

Dans certains modes de réalisation, le terminateur fort peut être une séquence provoquant un blocage des ribosomes, un site de clivage par une RNAse (c'est-à-dire une séquence de reconnaissance par des ribonucléases), une succession de codons rares (qui va faire intervenir une RNase), un terminateur comprenant au moins une structutre tige-boucle.

Dans un mode de réalisation particulier, un terminateur fort contient au moins une structure tige-boucle, c'est-à-dire une séquence d'ADN qui se replie pour former une stucture en épingle à cheveux. Une telle séquence d'ADN peut, par exemple, être constituée d'une séquence répétée inversée (ayant un fort delta G) suivie d'une série de thymines T (uracils U sur l'ARN transcrit). Lors de sa transcription en ARN, la séquence répétée inversée adopte une structure en tige et boucle qui provoque une pause de l'ARN polymérase. L'ARN transcrit n'est plus alors apparié au brin d'ADN matrice que par la séquence d'uridines qui suit. Ces interactions A-U sont faibles, et l'ARN synthétisé peut se détacher de sa matrice. La transcription s'arrête. Les structures en tige-boucle sont importantes dans les terminaisons de transcription intrinsèque (ou rho indépendantes) des procaryotes.

Ainsi, le terminateur en structure tige-boucle peut être le terminateur de transcription intrinsèque naturel d'une bactérie (en particulier de l'espèce ou souche de bactérie dans laquelle la construction génétique est destinée à être utilisée) ou un terminateur chimérique, ou bien encore une combinaison de tels terminateurs.

Dans certains modes de réalisation où la construction génétique est destinée à être utilisée dans *E. Coli,* le terminateur peut être choisi parmi le terminateur synthétique L3S2P56 (Ying-Jia Chen et al., Nature Methods, 2013, 10: 659-664) de séquence SEQ ID NO: 20: 
CTCGGACCAAATTTCGAAAAAAGACGCTGAAAAGCGTCTTTTTCGTTTGG
TCC
 le terminateur naturel ECK12002960 (Ying-Ja Chen et al., Nature Methods, 2013, 10: 659-664) de séquence SEQ ID NO: 21: 
TTCAGCCAAAAACTTAAGACCGCCGGTCTTGTCCACTACCTTGCAGTAATGC
GGTGGACAGGATCGGCGGTTTTCTTTTCTCTTCTCAA,
 le terminateur naturel de l'opéron rrnC de *E.coli* (Young, J. Biol. Chem., 1979, 254: 12725-12731) de séquence SEQ ID NO: 22: 
CCAGGCATCAAATAAAACGAAAGGCTCAGTCGAAAGACTGGGCCTTTCGTTT
TATCTGTTGTTTGTCGGTGAACGCTCTC
 ou une combinaison de ces terminateurs, comme par exemple, la séquence SEQ ID NO: 23:

CTCGGACCAAATTTTCGAAAAAAGACGCTGAAAAGCGTCTTTTTTCGTTTTGG
TCCTTCAGCCAAAAAACTTAAGACCGCCGGTCTTGTCCACTACCTTGCAGTAA
TGCGGTGGACAGGATCGGCGGTTTTCTTTTCTCTTCTCAACCAGGCATCAAAT
AAAACGAAAGGCTCAGTCGAAAGACTGGGCCTTTCGTTTTATCTGTTGTTTGT
CGGTGAACGCTCTC.

### Sites de Restriction

Comme indiqué plus haut, une construction génétique selon l'invention est conçue pour une utilisation dans une espèce ou souche de bactérie donnée. Cependant, une telle construction génétique peut être modifiée pour être transposable à d'autres espèces ou souches de bactérie en modifier la région promoteur, les gènes rapporteurs, le tag peptidique et/ou le terminateur. Cela peut par exemple être accompli par introduction de sites de restriction uniques, encadrant ces éléments.

Ainsi, préférablement, une construction génétique selon l'invention comprend des sites de restriction. Le terme "site de restriction" désigne une séquence particulière de nucléotides qui est reconnue par une enzyme de restriction comme un site de coupure dans la molécule d'ADN. Par exemple, des sites de restriction peuvent être placés au sein de la construction génétique pour permettre au système en son entier d'être transposé dans un vecteur, et/ou pour permettre la modification ou le remplacement du promoteur et/ou du premier gène rapporteur et/ou du deuxième gène rapporteur et/ou de l'étiquette peptidique et/ou du terminateur. Préférablement, les sites de restriction des éléments modifiables ou remplaçables (promoteur, premier gène rapporteur, deuxième gène rapporteur, étiquette peptidique et terminateur) sont tous uniques et différents les uns des autres pour que ces éléments puissent être modifiés ou remplacés spécifiquement et indépendamment les uns des autres.

Les sites de restriction et leur utilisation en biologie moléculaire sont connus dans l'art (www.neb.com/tools-and-resources/selection-charts/alphabetized-list-of-recognition-specificities) et l'homme du métier sait sélectionner des sites de restriction en fonction de l'utilisation désirée.

Des exemples de sites de restriction incluent, sans limitation, les sites de restriction reconnus par les enzymes BamHI, HindIII, KpnI, NdeI, ApaI, XbaI, BglII et EcoRI.

### Autres Séquences

D'autres séquences peuvent être présentes dans une construction génétique rapportrice selon l'invention.

Un exemple de telles séquences est au moins une séquence " amorce" qui permet d'amplifier la construction par PCR avant introduction dans un plasmide afin de pouvoir modifier des éléments par biologie moléculaire. Par exemple, une première séquence amplificatrice (GTAAAACGACGGCCAGT; SEQ ID NO: 24) peut être placé au début (5') de la construction et une deuxième séquence amplificatrice (GTCGTGACTGGGAAAAC; SEQ ID NO: 25) peut êre placée à la fin (3') de la construction.

### Exemple Particulier de Construction Génétique de Type Rapporteur

Les Inventeurs ont développé une construction génétique utilisable chez *E. coli,* caractérisée en ce que le promoteur est la séquence consensus décrite par Kanaya et Kudo (Nucleic Acids Symp., 1991, 25: 41-42), le premier gène rapporteur code pour mCherry, l'étiquette peptidique a la séquence ANDENYALAA (SEQ ID NO: 1), le deuxième gène rapporteur code pour la GFP, les sites d'initiation de la traduction sont constitués d'un site de fixation au ribosome de séquence AAGGAGA et de nucléotides supplémentaires de séquence TATACAT et le terminateur fort a la séquence SEQ ID NO: 19. La séquence de la construction génétique commence par la séquence amplificatrice SEQ ID NO: 24 et se termine par la séquence amplificatrice SEQ ID NO: 25.

La construction génétique développée par les Inventeurs comprend également des sites de restriction reconnus par les enzymes BamHI (ou XhoI) et HindIII (ou SaII, ou EcoRI) pour que le système en son entier puisse être introduit dans un vecteur; des sites de restriction reconnus par les enzymes BamHI et KpnI pour permettre la modification ou remplacement du promoteur; des sites de restriction reconnus par les enzymes KpnI et ApaI pour permettre la modification ou remplacement du gène codant pour mCherry; des sites de restriction reconnus par les enzymes ApaI et XbaI pour permettre la modification ou remplacement de l'étiquette peptidique; des sites de restriction reconnus par les enzymes XbaI et BglII pour permettre la modification ou remplacement du gène codant pour la GFP; et des sites de restriction reconnus par les enzymes BglII et EcoRI (ou SaII, ou HindIII) pour permettre la modification ou remplacement du terminateur.

La construction génétique développée par les Inventeurs a la séquence SEQ ID NO: 26 suivante:

GTAAAACGACGGCCAGTTAACTTATGCATCTCGAGGGATCCATTTAAATTGTT
GACAATTAATCATCGGCTCGTATAATGTGTGGAATTGTGAGCGGATAACAAT
TTGCTAGCGGTACCGAAGGAGATATACATATGGTGAGCAAGGGCGAGGAG
ATAACATGGCCATCATCAAGGAGTTCATGCGCTTCAAGGTGCACATGGAGGG
CTCCGTGAACGGCCACGAGTTCGAGATCGAGGGCGAGGGCGAGGGCCGCCC
CTACGAGGGCACCCAGACCGCCAAGCTGAAGGTGACCAAGGGTGGCCCCCTG
CCCTTCGCCTGGGACATCCTGTCCCCTCAGTTCATGTACGGCTCCAAGGCCTA
CGTGAAGCACCCCGCCGACATCCCCGACTACTTGAAGCTGTCCTTCCCCGAG
GGCTTCAAGTGGGAGCGCGTGATGAACTTCGAGGACGGCGGCGTGGTGACCG
TGACCCAGGACTCCTCCCTGCAGGACGGCGAGTTCATCTACAAGGTGAAGCT
GCGCGGCACCAACTTCCCCTCCGACGGCCCCGTAATGCAGAAGAAGACCATG
GGCTGGGAGGCCTCCTCCGAGCGGATGTACCCCGAGGACGGCGCCCTGAAGG
GCGAGATCAAGCAGAGGCTGAAGCTGAAGGACGGCGGCCACTACGACGCTG
AGGTCAAGACCACCTACAAGGCCAAGAAGCCCGTGCAGCTGCCCGGCGCCTA
CAACGTCAACATCAAGTTGGACATCACCTCCCACAACGAGGACTACACCATC
GTGGAACAGTACGAACGCGCCGAGGGCCGCCACTCCACCGGCGGCATGGAC
GAGCTGTACAAGGGGCCCGCAGCAAACGACGAAAACTACGCTCTCGCGGCTT
AATCTAGATTTAAGAAGGAGATATACATATGAGTAAAGGAGAAGAACTTTTC
ACTGGAGTTGTCCCAATTCTTGTTGAATTAGATGGTGATGTTAATGGGCACAA
ATTTTCTGTCAGTGGAGAGGGTGAAGGTGATGCAACATACGGAAAACTTACC
CTTAAATTTATTTGCACTACTGGAAAACTACCTGTTCCATGGCCAACACTTGT
CACTACTTTCGGGTATGGTGTTCAATGCTTTGCGAGATACCCAGATCATATGA
AACAGCATGACTTTTTCAAGAGTGCCATGCCCGAAGGTTATGTACAGGAAAG
AACTATATTTTTCAAAGATGACGGGAACTACAAGACACGTGCTGAAGTCAAG
TTTGAAGGTGATACCCTTGTTAATAGAATCGAGTTAAAAGGTATTGATTTTAA
AGAAGATGGAAACATTCTTGGACACAAATTGGAATACAACTATAACTCACAC
AATGTATACATCATGGCAGACAAACAAAAGAATGGAATCAAAGTTAACTTCA
AAATTAGACACAACATTGAAGATGGAAGCGTTCAACTAGCAGACCATTATCA
ACAAAATACTCCAATTGGCGATGGCCCTGTCCTTTTACCAGACAACCATTACC
TGTCCACACAATCTGCCCTTTCGAAAGATCCCAACGAAAAGAGAGACCACAT
GGTCCTTCTTGAGTTTGTAACAGCTGCTGGGATTACACATGGCATGGATGAAC
TATACAGATCTCTCGGACCAAATTTTCGAAAAAAGACGCTGAAAAGCGTCTT
TTTTCGTTTTGGTCCTTCAGCCAAAAAACTTAAGACCGCCGGTCTTGTCCACT
ACCTTGCAGTAATGCGGTGGACAGGATCGGCGGTTTTCTTTTCTCTTCTCAAC
CAGGCATCAAATAAAACGAAAGGCTCAGTCGAAAGACTGGGCCTTTCGTTTT
ATCTGTTGTTTGTCGGTGAACGCTGTCGACTAGTGAGCTCAAGCTTGAATTCC
GGAGTCGTGACTGGGAAAAC

### Vecteurs ou Plasmides

Une cassette d'expression selon l'invention est préférablement insérée dans un vecteur ou plasmide approprié. De nombreux vecteurs et plasmides peuvent être utilisés dans le contexte de la présente invention. Cependant, comme le sait l'homme du métier, le vecteur ou plasmide doit, soit être approprié pour l'utilisation dans l'espèce ou la souche de bactérie dans laquelle est le système rapporteur est destiné à être utilisé, soit être un vecteur ou plasmide à spectre large, par exemple un vecteur à spectre large adapté à l'utilisation chez les bactéries Gram- ou un vecteur à spectre large adapté à l'utilisation chez les bactéries Gram+.

On entend ici par "vecteur", une molécule d'ADN qui est indifféremment sous la forme de simple brin ou de double brin. Un vecteur recombinant selon l'invention est de préférence un vecteur plasmidique ou un vecteur d'intégration (ou vecteur intégratif). Dans certains modes de réalisation de l'invention, le vecteur est un plasmide. On entend ici par "plasmide", une molécule d'ADN circulaire double brin, qui possède obligatoirement une origine de réplication, afin qu'il puisse se répliquer de manière autonome dans la cellule et un gène de sélection pour qu'il ne soit pas perdu par l'organisme au fil des multiplications cellulaires.

Un vecteur/plasmide comprend généralement des séquences appropriées d'initiation et d'arrêt de la transcription. En outre, un vecteur/plasmide peut donc inclure au moins une origine de réplication fonctionnelle chez la bactérie à laquelle le vecteur est destiné, ainsi que, le cas échéant, des séquences nucléotidiques marqueurs de sélection (tels que les marqueurs de résistance aux antibiotiques Ampicilline, Kanamycine et Tetracycline ou des marqueurs d'auxotrophie pour éviter une synergie des deux antibactériens).

Un vecteur/plasmide utile dans le contexte de l'invention est, en particulier, un vecteur qui permet l'expression inductible des gènes de la construction génétique, c'est-à-dire qui permet l'induction spécifique de l'expression en réponse à la présence d'un composé particulier appelé inducteur. Ainsi, préférablement, un vecteur/plasmide utile dans le contexte de l'invention contient un promoteur fort (si supprimé de la cassette), aisément inductible et dont l'activité est négligeable en l'absence d'inducteur spécifique. Il porte éventuellement le gène régulateur permettant de contrôler l'expression de la cassette. Des exemples d'inducteurs appropriés à l'utilisation chez les bactéries incluent, sans limitation, l'arabinose qui est utilisée avec le régulateur araC et l'IPTG (isopropyl β-D-1-thiogalactopyranoside) qui est utilisé avec le répresseur LacI.

Dans certains modes de réalisation, le vecteur/plasmide contenant une construction génétique selon l'invention contient un promoteur inductible par l'IPTG. L'IPTG est souvent utilisé pour induire l'expression de protéines recombinantes placées sous le contrôle du répresseur lacI dans *E. coli.* Les bactéries portant un plasmide contenant le gène d'intérêt sont cultivées en l'absence d'IPTG, jusqu'à ce que la densité cellulaire atteigne un niveau suffisant. L'addition d'IPTG dans la culture permet d'induire la production de la protéine au moment désiré.

Certains des plasmides pET, qui sont commercialisés par Novagen, permettent l'expression de protéines recombinantes chez *E. coli.* Dans la construction réalisée par les présents Inventeurs, pET15 a été utilisé.

Des exemples de vecteurs ou plasmides à spectre large adaptés à l'utilisation chez les bactéries Gram- incluent, sans limitation, le vecteur pBBR1-MCS2 (Szpirer et al., J. Bacteriol., 2001, 183: 2101-2110; Michael E. Kovach, Gene, 1995, 166: 175-176)

Des exemples de vecteurs ou plasmides à spectre large adaptés à l'utilisation chez les bactéries Gram+ incluent, sans limitation, les vecteurs possédant une origine de réplication de pAM β, pE194, ou pT101.

### Préparation de Cassettes d'Expression et de Vecteurs

Une cassette d'expression ou un vecteur/plasmide selon l'invention peut être préparé par n'importe quelle méthode appropriée, la méthode d'obtention de la cassette ou du vecteur/plasmide n'étant pas un élément critique ou limitant de l'invention.

Des méthodes de préparation de telles constructions d'acide nucléique sont connues dans l'art et ont, par exemple, été décrites dans plusieurs ouvrages de référence comme Sambrook, Fritsch et Maniatis, "Molecular Cloning: A Laboratory Manual", 1989, Cold Spring Harbor Laboratory: Cold Spring Harbor, et Silhavy, Berman, et Enquist, "Experiments with Gene Fusions", 1984, Cold Spring Harbor Laboratory: Cold Spring Harbor; F.M. Ausubel et al., "Current Protocols in Molecular Biology", 1989, John Wiley & Sons: New York.

### Cellules Bactériennes Hôtes

L'invention se rapporte aussi aux cellules bactériennes hôtes comprenant une construction ou un vecteur/plasmide selon l'invention. On entend par "hôte cellulaire bactérien" ou " cellule bactérienne hôte", une cellule de bactérie qui a été transformée par une construction génétique ou un vecteur/plasmide selon l'invention.

L'hôte cellulaire bactérien peut être une cellule de n'importe quelle bactérie, en particulier de n'importe quelle bactérie pathogène pour l'homme. En particulier, l'hôte cellulaire bactérien peut être, sans limitation, une cellule de *Escherichia coli, Yersinia pestis, Vibrio cholerae, Shigella dysenteriae, Shigella flexneri, Klebsiella pneumoniae, Serratia marcescens, Enterobacter aerogenes, Clostridium tetani, Mycobacterium tuberculosis, Mycobacterium leprae, Corynebacterium diphtheriae, Chlamydia trachomati,Borrelia burdorferi,Streptococcus pneumoniae, Streptococcus pyogenes, Pseudomonas aeruginosa, Haemophilus influenzae, Bordetella pertussis, Bordetella parapertussis, Neisseria gonorrhoeae, Neisseria meningitides, Clostridium botulinum, Treponema pallidum, Staphylococcus aureus,* ou *Helicobacter pylori.*

La transformation de cellules bactériennes avec une construction génétique ou un vecteur/plasmide selon l'invention peut être réalisée par n'importe quelle technique appropriée connue de l'homme du métier. Des méthodes d'introduction de cassettes d'expression dans des cellules de bactéries ont été décrites, comme par exemple la transformation et la conjugaison. Voir, par exemple, Sambrook, Fritsch et Maniatis, "Molecular Cloning: A Laboratory Manual", 1989, Cold Spring Harbor Laboratory: Cold Spring Harbor.

On peut citer notamment les méthodes d'électroporation et de choc thermique. La technique d'électroporation consiste à placer les bactéries en solution en présence du vecteur/plasmide à des impulsions électriques provoquant des pores à travers la paroi et ainsi permettre l'entrée du vecteur/plasmide. Dans les méthodes de choc thermique, les bactéries et le vecteur/plasmide sont plongés dans une solution de chlorure de calcium, qui agit sur la paroi des bactéries en créant des orifices. On fait subir dans le même temps un choc thermique en plaçant l'ensemble sur de la glace afin de permettre au vecteur/plasmide d'entrer en contact avec la paroi puis le retour à une températion de 37°C afin d'assurer l'introduction de celui-ci dans la bactérie.

Préférablement, un hôte cellulaire bactérien comprenant une construction ou un vecteur/plasmide selon l'invention est "transformé de façon stable". Le terme "transformé de façon stable", tel qu'utilisé ici, désigne une cellule bactérienne dans lequel un acide nucléique exogène qui a été introduit par une méthode de transformation ou de conjugaison, est capable de réplication. La stabilité de la transformation est démontrée par la capacité de la cellule transformée à établir des lignées cellulaires ou des clones comprenant une population de cellules filles qui contiennent elles aussi l'acide nucléique exogène.

Le succès de la transformation d'une cellule bactérienne peut être évalué de façon préliminaire visuellement lorsque la cassette d'expression ou le vecteur/plasmide utilisé contient un gène marqueur.

### II - Applications du Système Rapporteur

Les systèmes rapporteurs décrits ici (vecteurs/plasmides comprenant une construction génétique selon l'invention) trouvent application en tant qu'outils de recherche fondamentale pour étudier la *trans*-traduction, et dans des méthodes de criblage pour identifier des composés capables d'inhiber la *trans*-traduction. L'expression "capable d'inhiber la *trans*-traduction", telle qu'utilisée ici pour définir ou caractériser un composé, désigne un composé qui réduit, freine, empêche ou prohibe, de manière totale ou partielle, l'activité *trans*-traductionnelle, et ce quel que soit le mécanisme d'action du composé pour inhiber la *trans*-traduction.

### Criblage

De manière générale, une méthode de criblage selon l'invention comprend une étape consistant à incuber une cellule bactérienne transformée par une construction génétique ou un vecteur/plasmide selon l'invention avec un composé à tester (ou composé test) et une étape consistant à détecter la présence ou l'absence de la protéine codée par le premier gène rapporteur et la présence ou l'absence de la protéine codée par le deuxième gène rapporteur.

### Transformation d'une Cellule Bactérienne

Les cellules bactériennes qui peuvent être utilisées dans un procédé de criblage selon l'invention incluent toute cellule de bactérie Gram+ ou Gram- pour laquelle un système rapporteur selon l'invention a été développé. Le procédé de criblage peut, en particulier, être mis en oeuvre avec des cellules de bactéries pathogènes pour l'homme comme celles décrites haut.

Avant de commencer le criblage, la viabilité des cellules peut être déterminée, par exemple en utilisant une méthode standard.

Comme indiqué plus haut, la transformation d'une cellule bactérienne avec un vecteur ou plasmide comprenant une construction génétique selon l'invention peut être effectuée par n'importe quelle méthode appropriée, comme une méthode d'électroporation ou une méthode de choc technique.

### Composés Tests

Dans un procédé de criblage selon l'invention, tout type de composés peut être testé. Ainsi, un composé test peut être un produit naturel ou un produit synthétique; il peut être une molécule unique ou bien un mélange ou un complexe de différentes molécules. Dans certains modes de réalisation, un composé test appartient à une chimiothèque (c'est-à-dire une banque de molécules). Les chimiothèques peuvent contenir de plusieurs dizaines à plusieurs millions de composés chimiques. Des chimiothèques de composés naturels sous la forme d'extraits bactériens ou fongiques, ou sous la forme d'extraits de plantes sont disponibles par exemple chez Pan Laboratories (Bothell, WA) ou MycoSearch (Durham, NC). Des chimiothèques de composés synthétiques sont également commercialement disponibles, par exemple, chez Comgenex (Princeton, NJ), Brandon Associates (Merrimack, NH), Microsource (New Milford, CT), et Aldrich (Milwaukee, WI) ou chez des grandes sociétés chimiques comme Merck, Glaxo Welcome, Bristol-Meyers-Squibb, Novartis, Monsanto/Searle, et Pharmacia UpJohn.

Les composés test peuvent appartenir à n'importe quelle classes de molécules, comme les protéines, les peptides, les peptidomimétiques, les peptoïdes, les saccharides, les stéroïdes, etc... Les composés test peuvent également être des "small molecules" ou molécules de petits poids molécules, généralement compris entre envion 50 et environ 2500 Daltons, comme par exemple entre 500 et 700 Daltons, et moins de 350 Daltons.

Dans certains modes de réalisation, les composés à tester sont connus pour démontrer une activité antibactérienne mais leur mécanisme d'action n'est pas connu.

Dans d'autres modes de réalisation, les composés à tester sont susceptibles de présenter une activité anti-*trans*-traduction, et l'on souhaite tester si cette activité est spécifique ou si elle est accompagnée par une activité inhibitrice des protéases.

L'incubation dans une méthode de criblage selon l'invention peut être réalisée par n'importe quelle méthode appropriée. Ainsi, le composé test peut être incubé avec les cellules bactériennes sur ou dans tout support approprié et notamment sur une plaque, dans un tube ou une flasque, sur une membrane, sur un support gélifié, ou sur un biofilm, etc.... Dans certains modes de réalisation, l'incubation est réalisée dans une plaque multipuits, ce qui permet de conduire, en parallele, des essais nombreux et variés. Parmi les supports typiques, on touve les plaques de microtitration et plus particulièrement les plaques de 12, 24, 48, 96, 384 puits (ou plus), qui sont faciles à manipuler.

Selon le support et la nature du composé test, des quantités variables de cellules peuvent être utilisées lors de la mise en oeuvre des procédés décrits ici. De manière classique, 10³ à 10⁶ cellules sont mises à incuber avec un composé test, et de manière préférentielle entre 10⁴ et 10⁵ cellules.

Dans un procédé de criblage selon l'invention, l'incubation de cellules bactériennes transformées par une construction génétique ou un vecteur/plasmide selon l'invention avec un composé test peut être effectuée dans n'importe quelles conditions appropriées de culture de cellules bactériennes (température, humidité, milieu de culture, etc...). Les conditions de culture de bactéries sont connues dans l'art. La concentration en composé test pendant l'incubation peut être ajustée selon le type de composé (sa toxicité, sa capacité de pénétration cellulaire, etc...), le nombre de cellules, la longueur de la période d'incubation, etc. Généralement, dans une méthode de criblage, les cellules sont exposées à des concentrations de composé test allant d'environ 1 fM à environ 10 mM. Préférablement, les concentrations utilisées sont comprises entre environ 10 pM et environ 100 µM. Il est bien sûr possible de tester d'autres concentrations sans dévier de la présente invention. Chaque composé peut, de plus, être testé, en parallèle, à différentes concentrations. L'incubation peut être maintenue pour n'importe quelle durée appropriée, par exemple entre quelques minutes et plusieurs heures ou jours, particulièrement entre 5 et 72 heures, généralement entre 12 et 48 heures.

Un procédé de criblage selon l'invention peut également être utilisé pour rechercher des combinaisons d'agents thérapeutiques, par exemple pour mettre en évidence les effets synergiques d'un agent thérapeutique connu avec un composé test. Dans ces modes de réalisation de l'invention, l'étape d'incubation dans un procédé de criblage selon l'invention est effectuée en présence d'une combination d'agents thérapeutiques connus ou d'une combination d'un ou plusieurs agents thérapeutiques connus avec un composé test.

### Détection des Protéines Codées par les Gènes Rapporteurs

Dans un procédé de criblage selon l'invention, l'étape de détection de la présence ou de l'absence des protéines codées par les gènes rapporteurs de la construction génétique peut se faire par détection ou mesure de leur activité enzymatique (si ces protéines sont des enzymes) ou de leur fluroescence (si ces protéines sont des protéines fluorescentes).

Dans certains modes de réalisation où le premier gène rapporteur code pour une première protéine fluorescente et le deuxième gène rapporteur code pour une deuxième protéine fluroescente, l'étape de détection comprend la quantification de la fluorescence émise par première protéine fluorescente et la quantification de la fluorescence émise par la deuxième protéine fluroescente. La quantification ou mesure de fluorescence peut être effectuée par n'importe quelle technique de fluorescence: microscopie de fluorescence, spectrocopie de fluorescence, etc... Ainsi, par exemple, les mesures de fluorescence peuvent être réalisées par microscopie de fluorescence par observation entre lame et lamelle d'un d'échantillon à partir d'une culture liquide. Il est également possible d'observer la fluorescence des bactéries en réalisant un état frais à partir d'une colonie sur gélose. Enfin, il est possible de suivre en parallèle la croissance bactérienne et la fluorescence du système rapporteur en réalisant la culture bactérienne directement sous le microscope, en utilisant des pads de gel d'agarose (supports gélifiés fixant les bactéries). Après acquisition des images, la détermination de fluorescence pour être réalisé par traitement information en utilisant des logiciels tels que PCI ou ImageJ (Ramadoss et al., P.N.A.S USA, 2016, 110: 10282-10287).

### Identification de Composés Inhibiteurs de la Trans-Traduction

Dans un procédé de criblage selon l'invention, un composé test est identifié comme un composé capable d'inhiber de façon spécifique la *trans*-traduction (c'est-à-dire sans inhiber l'activité des protéases) si seule la protéine codée par le deuxième gène rapporteur est détectée. Un composé test est identifié comme un composé capable d'inhiber de façon spécifique les protéases (c'est-à-dire sans inhiber la trans-traduction) si la protéine codée par le premier gène rapporteur et la protéine codée par le deuxième gène rapporteur sont détectées en quantité égale. Un composé test est identifié comme un composé capable d'inhiber les protéases et la trans-traduction si les protéines codées par les premier et deuxième gènes rapporteurs sont détectées et si la protéine codée par le deuxième gène rapporteur est détectée en quantité plus élevée que la protéine codée par le premier gène rapporteur.

Ainsi, pour une construction génétique comprenant un premier gène rapporteur codant pour une première protéine fluorescente et un deuxième gène rapporteur codant pour une deuxième protéine fluorescente, le composé test est identifié comme un composé capable d'inhiber la *trans*-traduction de manière spécifique si la fluorescence de la deuxième protéine fluorescente (par exemple la fluorescence verte de GFP) est détectée mais la fluorescence de la première protéine fluorescente (par exemple la fluorescence rouge de mCherry) n'est pas détectée. Le composé test est identifié comme un composé capable d'inhiber de manière spécifique les protéases si la fluorescence de la première protéine fluroescente (par exemple la fluorescence rouge de mCherry) et la fluorescence de la deuxième protéine fluorescente (par exemple la fluorescence verte de GFP) sont détectées, et si les fluorescences mesurées correspondent à des quantités égales de GFP et de mCherry. Le composé test est identifié comme un composé capable d'inhiber les protéases et la *trans*-traduction si la fluorescence de la première protéine fluroescente (par exemple la fluorescence rouge de mCherry) et la fluorescence de la deuxième protéine fluorescente (par exemple la fluorescence verte de GFP) sont détectées, et si les fluorescences mesurées correspondent à une quantité de la deuxième protéine fluorescente (par exemple la GFP) plus élevée que la quantité de la première protéine fluorescence (par exemple mCherry). Les rapports des mesures de fluorescence des deux protéines permet de définir les activités relatives du composé test.

### Développement d'Antibiotiques à Large Spectre

Un procédé de criblage selon l'invention a pour but d'identifier des composés susceptibles de présenter un intérêt en tant qu'antibiotiques.

Une méthode de criblage selon l'invention permet, en particulier, d'identifier des composés qui ont une action *anti*-trans-traduction spécifique ou qui présente une action *anti*-trans-traduction majoritaire comparée à leur activité inhibitrice de protéases. Un test de criblage selon l'invention peut être suivi par d'autres essais, par exemple par un ou plusieurs tests de criblage selon l'invention conduits sur d'autres espèces de bactéries. Alternativement ou additionnellement, un test de criblage selon l'invention peut être suivi par des études de toxicité *in vivo* sur des modèles cellulaires ou sur des modèles animaux d'infections bactériennes. L'activité antibactérienne peut être vérifiée par l'étude des croissances bactériennes, par la réalisation d'antibiogrammes et l'étude des Concentrations Minimales Inhibitrices/Bactéricides (CMI/CMB).

Lorsqu'un composé test a été identifié comme présentant une action *anti*-trans-traduction spécifique ou majoritaire, des études de relations structure-activité peuvent être menées dans le but d'identifier de nouveaux squelettes antibiotiques présentant des propriétés améliorées comparées au composé test identifié et de développer de nouvelles stratégies thérapeutiques au-delà des multirésistances bactériennes connues.

L'invention concerne également les composés identifiés à l'aide d'une méthode de criblage selon l'invention ou les dérivés de ceux-ci, ainsi que toute composition pharmaceutique comprenant au moins un composé identifié par une méthode de criblage selon l'invention ou un dérivé de celui-ci. Une composition pharmaceutique selon l'invention comprend au moins un composé identifié à l'aide d'une méthode de criblage selon l'invention ou un dérivé de delui-ci et au moins un véhicule ou excipient physiologiquement acceptable. Dans le contexte de la présente invention, on entend par "véhicule ou excipient physiologiquement acceptable" tout milieu ou additif qui n'interfère pas avec l'efficacité de l'activité biologique du principe actif et qui n'est pas excessivement toxique pour le patient ou sujet, aux concentrations auxquelles il est administré. Préférablement, le véhicule ou excipient physiologiquement acceptable est approprié pour l'utilisation pharmaceutique humaine.

L'invention concerne aussi l'utilisation de ces composés, dérivés et/ou compositions pharmaceutiques comme antibiotiques. La présente invention se rapporte également à une méthode de traitement d'une maladie bactérienne comprenant une étape consistant à administrer à un patient ou un sujet, une quantité efficace d'un composé ou d'un dérivé ou d'une composition pharmaceutique comprenant un tel composé ou dérivé. Cette méthode peut, en particulier, être utilisée pour le traitement d'une infection bactérienne, telle qu'une infection causée par une bactérie pathogène Gram+ ou une bactérie pathogène Gram-. Dans le contexte de la présente invention, on entend par "traitement" une méthode qui a pour but (1) de retarder ou prévenir le début d'une maladie ou d'une condition clinique; (2) de ralentir ou d'arrêter la progression, l'aggravation ou la détérioration des symptômes de la maladie; (3) d'apporter des améliorations des symptômes de la maladie; et/ou (4) de guérir la maladie. Un traitement peut être administré avant le début de la maladie pour une action préventive, ou il peut être administré après initiation de la maladie, pour une action thérapeutique. Le terme "sujet" désigne ici un mammifère, alors que le terme "patient" est préférablement utilisé lorsque le sujet est un être humain. Un composé identifié par une méthode de criblage selon l'invention (ou un dérivé de celui-ci ou une composition pharmaceutique comprenant un tel composé ou dérivé) peut être administré par n'importe quelle voie d'administration appropriée (orale, parentérale, rectale, pulmonaire, nasale, cutanée, transdermique, mucosale, etc) et peut être locale ou systémique. La voie optimale d'administration peut être déterminée en fonction de la nature et/ou de la localisation de l'infection bactérienne.

Les infections bactériennes qui peuvent être traitées par administration d'un composé identifié par une méthode de criblage selon l'invention ou d'un dérivé de celui-di, incluent, sans limitation, les infections urinaires bactériennes, les infections cutanées bactériennes, les infections intestinales bactériennes, les infections pulmonaires bactériennes, les infections ostéo-articulaires bactériennes, les infections génitales bactériennes, les infections dentaires bactériennes, la septicémie, les infections nosocomiales, les méningites bactériennes, les gastro-entérites bactériennes, les endocardites, les pneumonies endocardites, les angines bactériennes, les otites bactériennes, les salmonelloses, etc. En particulier, un composé identifié par une méthode de criblage selon l'invention, ou un dérivé de celui-ci peut être utilisé pour traiter la tuberculose (qui est causée par la bactérie *Mycobacterium tuberculosis*), la lèpre (causée par *Mycobacterium leprae),* la peste (causée par *Yersinia pestis*), le choléra (causé par *Vibrio cholerae),* le tétanos (causé par *Clostridium tetani*)*,* le botulisme (causé par *Clostridium botulinum),* la diphtérie (causée par *Corynebacterium diphtheriae*), la dysentrie (causée par *Shigella dysenteriae*)*,* la chlamydia (causée par *Chlamydia trachomatis*)*,* la gonorrhée (causée par *Neisseria gonorrhoeae),* la méningite (causée par *Neisseria meningitides* ou par *Haemophilus influenzae*)*,* la syphilis (causée par *Treponema pallidum),* la maladie de lyme (causée par *Borrelia burdorferila* cocheluche (causée par *Borrelia parapertussis*), l'ulcère gastro-duodénal (causé par *Helicobacter pylori),* les pneumonies (causées par *Streptococcus pneumoniae* ou par *Streptococcus pyogenes*), les infections causées par *Staphylococcus aureus* comme le syndrome du choc toxique, et les infections opportunistes et nocosomiales causées par *Pseudomonas aeruginosa, Klebsiella pneumoniae, Serratia marcescens* ou *Enterobacter* aerogenes.

### III - Kits

La présente invention vise également des kits comprenant du matériel utile pour la réalisation d'une méthode selon l'invention. En particulier, la présente invention concerne des kits pour l'étude fondamentale de l'activité *trans*-traductionnelle et des kits pour le criblage de composés capables d'inhiber la *trans*-traduction chez au moins une espèce ou souche de bactérie. Ainsi, dans certains modes de réalisation, un kit selon l'invention peut être conçu pour contenir une construction génétique (sous forme de vecteur/plasmide ou non) destinée à être utilisée dans différentes espèces ou souches de bactéries Gram-. Dans d'autres modes de réalisation, un kit selon l'invention peut être conçu pour contenir une construction génétique (sous forme de vecteur/plasmide ou non) destinée à être utilisée dans différentes espèces ou souches de bactéries Gram+.

De manière générale, un kit selon l'invention comprend une construction génétique ou un vecteur/plasmide comprenant la construction génétique.

Dans certains modes de réalisation, un kit peut également comprendre un inducteur spécifique du promoteur fort du vecteur/plasmide, par exemple l'IPTG.

Dans certains modes de réalisation où la construction génétique a été conçue pour être transposable à l'utilisatoin dans d'autres espèces ou souches de bactéries, un kit selon l'invention peut comprendre des enzymes de restriction permettant au système rapporteur en son entier d'être transposé dans un autre plasmide, et/ou des enzymes de restriction permettant le remplacement du promoteur et/ou du premier gène rapporteur et/ou du deuxième gène rapporteur et/ou de l'étiquette peptidique et/ou du terminateur fort. Des exemples de telles enzymes de restriction sont les enzymes BamHI, HindIII, KpnI, NdeI, ApaI, XbaI, BglII et EcoRI. Le kit peut également comprendre un ou plus des éléments suivants: un ou plusieurs plasmides différents, un ou plusieurs promoteurs différents, un ou plusieurs premiers gènes rapporteurs différents, un ou plusieurs deuxièmes gènes rapporteurs différents, une ou plusieurs étiquettes peptidiques différentes, et un ou plusieurs terminateurs forts différents.

Dans certains modes de réalistion, un kit peut en outre comprendre des cellules bactériennes destinées à être transformées par la construction génétique ou le vecteur/plasmide contenu dans le kit. Alternativement, un kit peut comprendre des cellules bactériennes déjà transformées.

Un kit selon l'invention peut en outre comprendre des réactifs ou solutions pour la mise en oeuvre d'une méthode de criblage selon l'invention, par exemple des réactifs ou solutions pour transformer des cellules bactériennes avec une construction ou un vecteur/plasmide selon l'invention, des réactifs ou solutions pour cultiver de telles cellules en solution liquide, sur gel ou en biofilm, etc.... Des protocoles pour utiliser ces réactifs et/ou solutions peuvent également être inclus dans le kit.

Les différents composants du kit peuvent être fournis sous forme solide (par exemple sous forme lyophilisée) ou sous forme liquide. Un kit peut éventuellement comprendre un récipient contenant chacun des réactifs ou solutions, et/ou des récipients pour réaliser certaines étapes du procédé de criblage de l'invention.

Un kit selon l'invention peut également comprendre des instructions pour mettre en oeuvre une méthode de criblage selon l'invention.

Un kit selon l'invention peut également comprendre une notice sous la forme prescrite par une agence gouvernementale régulant la préparation, la vente et l'utilisation de produits biologiques.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevet, tous les brevets et toutes autres références mentionnés ici sont incorporés par référence.

Les exemples suivants et les figures sont présentés pour illustrer certains modes de réalisation des procédures décrites ci-dessus et ne doivent en aucun cas être considérés comme une limite à la portée de l'invention.

### Exemples

Les exemples suivants décrivent certains modes de réalisation de la présente invention. Cependant, il est entendu que les exemples ne sont présentés qu'à titre illustratif seulement et ne limitent en aucun cas la portée de l'invention.

### Matériels et Méthodes

*Transformation de Cellules Bactériennes avec un Plasmide Rapporteur par une Méthode d'Electroporation.* Les bactéries électro-compétentes ainsi que le plasmide à introduire dans les bactéries sont mis en contact dans une cuve d'électroporation. Le mélange est soumis à un choc électrique. Un milieu riche est alors ajouté au mélange, les bactéries sont incubées pour relancer la croissance puis étalées sur des boites de milieux adaptés pour la sélection.

*Suivi de Croissance.* Les pré-cultures BW25113 et la construction génétique (pRV2) en phase stationnaire (culture en milieu LB toute la nuit) sont diluées dans du milieu LB pour une Densité Optique DO₆₀₀ = 0,025. Les cellules sont cultivées, en incubateur à +37°C sous agitation à 190 rpm jusqu'à une densité optique DO = ∼0,1. A ce moment, l'IPTG est ajouté aux cellules pour une concentration finale de 1 mM. La densité optique est déterminée par spectrophotomètrie.

*Analyses Fluorimétrique.* Après 45 minutes d'induction à l'IPTG, 100 µL de la suspension cellulaire est prélevée afin de détecter la fluorescence de la GFP et du mCherry. Des prélèvements sont ensuite effectués environ toutes les heures pour effectuer une nouvelle mesure. Le rapport des fluorescences a été calculé en divisant l'intensité de fluorescence verte (GFP) par l'intensité de fluorescence rouge (mCherry). La correction du bruit de fond a été normalisé par rapport à la souche de référence "BW22513ΔClp" et reportée sur un graphique. Pour la GFP, la longueur d'onde excitation utilisée est 485 nm et la longueur d'onde d'émission est 535 nm, et le temps d'exposition utilisé est de 0,06 seconde. Pour la mCherry, la longueur d'onde excitation utilisée est 550 nm et la longueur d'onde d'émission est 610 nm, et le temps d'exposition utilisé est de 1,5 seconde. La fluorescence a été mesurée sur le fluorimètre Twinkcle LB 970 de Berthold Company

*Suivi de Croissance.* La méthode de lecture de la Densité Optique (DO) (600 nm) sur plaque est similaire à la méthode de lecture de fluorescence (une seule plaque peut être utilisée pour les mesures de fluorescence et le suivi de croissance). L'utilisation de cette méthode pour le suivi de croissance a été confirmée par une méthode conventionnelle afin de confirmer que la DO mesurée correspond bien au nombre de UFC.

*Lecture par Microscopie de Fluorescence.* La lecture du système rapporteur peut-être réalisée par microscopie de fluorescence par observation entre lame et lamelle de 5 µL d'échantillon à partir d'une culture liquide. Il est également possible d'observer la fluorescence des bactéries en réalisant un état frais à partir d'une colonie sur gélose. Enfin, il est possible de suivre en parallèle la croissance bactérienne et le système rapporteur en réalisant la culture bactérienne directement sous le microscope, en utilisant des pads de gel d'agarose (supports gélifiés fixant les bactéries) (Young et al., Nature Methods, 2012, 7: 80-88). Après acquisition des images, la détermination de fluorescence pour être réalisé par traitement information en utilisant des logiciels tels que PCI ou ImageJ (Ramadoss et al., P.N.A.S USA, 2016, 110: 10282-10287).

### Résultats

### Système Rapporteur de l'Activité Trans-Traductionnelle dans E. Coli

Le système rapporteur, développé par les présents Inventeurs et construit par clonage génétique, est présenté sur la Figure 1(A). Il permet de mesurer l'activité de *trans*-traduction mais également de suivre simultanément l'activité des protéases impliquées dans la dégradation des protéines marquées par l'ARNtm de *E. Coli.*

Dans la construction génétique, deux gènes rapporteurs ont été clonés en opéron, le gène codant mCHerry (qui fluoresce dans le rouge) et le gène codant pour la GFP (qui fluoresce dans le vert). Cette construction fonctionne comme indiqué sur la Figure 1(B) (voir également la Figure 3(A)-(C)).

Le premier gène codant pour mCherry (qui fluoresce dans le rouge) est lié en 3' à une séquence codant pour une étiquette peptidique identidque à celle ajoutée par l'ARNtm de *E. Coli.* En l'abscence de tout inhibiteur, la construction protéique corresponsant, nommée ici "mCherry_tag", est reconnue et dégradée par les protéases spécifiques de la *trans*-traduction. Aucune fluorescence de mCherry n'est alors observable. En présence d'un inhibiteur de ces protéases, mCherry_tag n'est pas dégradé et s'accumule dans les cellules (transformées avec la construction), conduisant à une fluorescence rouge. En présence d'un inhibiteur de la *trans*-traduction, mCherry_tag est reconnu et dégradé par les protéases spécifiques de la *trans*-traduction. Aucune fluorescence de mCherry n'est alors observable.

Le second gène rapporteur, qui code pour la GFP (qui fluoresce dans le vert) est lié en 3' à un terminateur fort sous forme de 3 tiges boucles à la place du codon stop. Cette partie de la construction conduit à la transcription d'un ARNm GFP non-stop qui est bloqué par les ribosomes, entrainant l'intervention de la *trans*-traduction. En l'abscence de tout inhibiteur, la protéine GFP bloquée se voit rajouter, par l'ARNtm, une étiquette peptidique qui est reconnue par des protéases spécifiques de la trans-traduction. La GFP taguée est donc dégradée. Aucune fluorescence de la GFP n'est observable. En présence d'un inhibiteur de ces protéases, la GFP taguée n'est pas dégradée et s'accumule dans les cellules (transformées avec la construction), conduisant à une fluorescence verte. En présence d'un inhibiteur de la *trans*-traduction, l'ARNtm ne peut pas ajouter une étiquette spécifique à la GFP. Cependant, la GFP est "sauvée" par d'autres mécanimes secondaires (ArfA et ArfB), sans toutefois être étiquetée et n'est donc pas dégradée par les protéases spécifiques de la *trans*-traduction. La GFP s'accumule donc dans les cellules (transformées avec la construction), conduisant à une fluorescence verte.

Ainsi, lorsque la construction génétique complète est utilisée dans une cellule bactérienne en l'absence de tout inhibiteur, aucune fluorescence n'est observée. En présence d'un inhibiteur des protéases, une fluorescence rouge et une fluorescene verte sont observée. En présence d'un inhibiteur spécifique de la *trans*-traduction (c'est-à-dire en l'absence d'une activité inhibitrice de protéases), seule une fluorescence verte est observée.

***Vecteur Rapporteur de trans***-***traduction chez E. coli.*** La cassette reportrice de l'activité de *trans*-traduction peut être clonée dans une multitude de vecteurs. Les Inventeurs ont cloné la cassette dans un vecteur pET-15b utilisé classiquement chez *E.coli* (voir Figure 2(A)). Ce vecteur est en faible nombre de copies (∼20 copies/bactérie), possède un gène de résistance à l'ampicilline utilisé comme pression de sélection pour le maintien du plasmide dans la bactérie. L'expression du système Rouge/Vert est sous le contrôle d'un promoteur fort (tac) et sous induction maitrisée *via* le gène *LacI,* qui permet de moduler cette induction en utilisant de l'IPTG (isopropyl β D-galactopyranoside).

### Vérification de l'Activité du Rapporteur de l'Activité Trans-Traductionnelle

En l'absence d'inhibiteurs connus de la *trans*-traduction, les Inventeurs ont utilisé plusieurs souches bactériennes mutées pour vérifier l'efficacité du système rapporteur. La délétion des gènes *Ssra* (codant pour l'ARNtm) ou *Smpb* (codant pour la protéine SmpB) permet de vérifier l'activité du rapporteur de la *trans*-traduction. La délétion du gène *ClpP* (codant pour la protéase la plus impliquée dans la dégradation des protéines marquées par tmRNA) permet d'étudier la voie de dégradation des protéases.

Pour chaque test réalisé, un suivi de croissance est également effectué par mesure de la densité optique à 600 nm, également en microplaques. Des mesures du nombre d'unité formant colonie (UFC) et des comparaisons avec des cultures en tubes permettent de valider l'utilisation du système rapporteur en condition de culture en microplaque.

Les résultats obtenus sont présentés dans la Figure 3. Ces résultats correspondent exactement à ceux attendus: en l'absence de *trans*-traduction, une fluorescence verte est observée; en l'abscence des protéases Clp, une fluorescence majoritairement rouge est observée avec également une fluorescence dans le vert résultant de la non-dégradation des GFP marqués par l'ARNtm. Ces résultats ont également été confirmés en restaurant, à l'aide d'un second plasmide, la présence ode la protéine SmpB dans une souche qui en était nativement dépourvue. L'activité *trans*-traductionnelle est alors rétablie et une diminution de la fluorescence verte a été observée.

L'observation de la fluorescence verte dans la souche BWΔClpP confirme que les protéases Clp jouent un rôle prépondérant dans la dégradation des protéines marquées par tmRNA et justifie l'utilisation de ce rapporteur de l'activité des protéases comme contrôle interne de l'activité *trans*-traductionelle. Ces résultats ont également été confirmés en restaurant, à l'aide d'un second plasmide, la présence de la protéine SmpB dans une souche qui en était nativement dépourvue. L'activité *trans*-traductionelle est alors rétablie et une diminution la fluorescence verte a été observée.

### Transposabilité du Rapporteur à d'Autres Espèces/Souches Bactériennes

Dans l'objectif de rendre le système transposable à d'autres espèces/souches bactériennes, les Inventeurs ont étudié la spécificité de l'étiquette ajoutée par la *trans-*traduction. Pour cela, ils ont remplacé l'étiquette du mCherry *d'Escherichia coli* (ANDENYALAA - SEQ ID NO: 1) par celui de *Staphylococcus aureus* (GKSNNNFAVAA - SEQ ID NO: 18). Dans *E. coli* WT, aucune fluorescence n'a été observée, ce qui indique que la protéine mCHERRY_e.coli-étiquetée (ANDENYALAA) est bien dégradée par les protéases. Ce résultat est confirmé par l'apparition de la fluorescence rouge dans la souche délétée pour Clp (BWΔClpP). Pour la protéine mCHERRY_s.aureus-étiquetée (GKSNNNFAVAA) dans *E. coli* WT, une faible fluorescence rouge a été observée, montrant que l'étiquette n'est que faiblement reconnue par les protéases. Il est donc important d'adapter l'étiquette selon l'espèce bactérienne. Pour cette raison, lors de la conception génétique du plasmide, des sites reconnus par des enzymes de restriction ont été insérés de part et d'autre du tag afin de faciliter son changement (Voir Figure 2(A)).

Afin d'adapter le système à d'autres bactéries Gram négatif, les Inventeurs ont cloné la construction génétique du système Rouge/Vert dans le vecteur pBBR1-MCS2 à large spectre (Kovach, Gène, 1995 166: 175-176). Le système obtenu est présenté sur la Figure 2(B). Les Inventeurs ont utilisé l'avantage du large spectre pour réaliser le clonage et vérifier son efficacité chez *E.coli.* Puis le plasmide a été transféré, par conjugaison, chez *Rhizobium melitoti* (une espèce à croissance lente utilisée comme modèle pour l'étude de l'espèce *Brucella* qui est pathogène pour l'homme et inscrite sur la liste des agents potentiels de bioterrorisme, et qui est dépourvue des mécanismes de secours ArfA et ArfB). La même démarche sera réalisée chez des bactéries à Gram positif (par exemple *B. subtilis, Staph. aureus*).

## Revendications

1. Construction génétique destinée à être utilisée dans une espèce ou souche de bactérie et comprenant:
- la séquence d'un premier gène rapporteur,
- la séquence d'un deuxième gène rapporteur, et
- la séquence d'un promoteur,
la construction génétique étant **caractérisée en ce que**:
- les premier et deuxième gènes rapporteurs sont différents et se trouvent sous le contrôle du promoteur,
- la séquence du premier gène rapporteur est liée de façon opérationnelle:
- en 5': à un site d'initiation de traduction, et
- en 3': à une séquence codant pour une étiquette peptidique et terminée par un codon stop, l'étiquette peptidique étant dégradée par les protéases spécifiques de la *trans*-traduction de l'espèce ou souche de bactérie dans laquelle la construction génétique est destinée à être utilisée,
- la séquence du deuxième gène rapporteur est liée de façon opérationnelle:
- en 5': à un site d'initiation de traduction, et
- en 3': à la séquence d'un terminateur fort, et
- le site d'initiation de traduction en 5' de la séquence du premier gène rapporteur et le site d'initiation de traduction en 5' de la séquence du deuxième gène rapporteur sont identiques.

2. Construction génétique selon la revendication 2, **caractérisée en ce que** les permier et deuxième gènes rapporteurs sont des gènes rapporteurs codant pour des protéines ayant une activité quantifiable, notamment codant pour des protéines fluorescentes.

3. Construction génétique selon la revendication 1 ou la revendication 2, **caractérisée en ce que** le site d'initiation de traduction comprend une séquence correspondant à un site de fixation au ribosome.

4. Construction génétique selon l'une quelconque des revendications 1 à 3, **caractérisée en ce que** le terminateur fort est une succession de codons rares, une séquence de reconnaissance par des ribonucléases, une séquence de blocage des ribosomes, ou un terminateur comprenant au moins une structure en tige-boucle.

5. Construction génétique selon l'une quelconque des revendications 1 à 4, **caractérisée en ce qu'**elle comprend en outre des sites de restriction permettantà la construction génétique d'être insérée dans un plasmide et des sites de restrictions permettant la modification ou le remplacement d'au moins un élément choisi parmi le promoteur, le premier gène rapporteur, le deuxième gène rapporteur, l'étiquette peptidique, et le terminateur fort.

6. Construction génétique selon la revendication 5, **caractérisée en ce que** les sites de restriction sont reconnus par les enzymes choisis parmi BamHI, HindIII, KpnI, NdeI, ApaI, XbaI, BglII, et EcoRI.

7. Construction génétique selon l'une quelconque des revendications 1 à 6, **caractérisée en ce qu'**elle est destinée à être utilisée dans *E. coli ,* **en ce qu'**elle est transposable à d'autres bactéries Gram- et **en ce que** sa séquence est la séquence SEQ ID NO: 26.

8. Vecteur ou plasmide comprenant une construction génétique selon l'une quelconque des revendications 1 à 7.

9. Vecteur ou plasmide selon la revendication 8, **caractérisé en ce qu'**il comprend un promoteur inductible.

10. Vecteur ou plasmide selon la revendication 9, **caractérisé en ce que** le promoteur est inductible par un inducteur chimique, préférablement l'IPTG.

11. Cellule bactérienne hôte comprenant une construction génétique selon l'une quelconque des revendications 1 à 7 ou un vecteur ou plasmide selon l'une quelconque des revendications 8 à 10.

12. Utilisation d'une construction génétique selon l'une quelconque des revendications 1 à 7 ou d'un vecteur ou plasmide selon l'une quelconque des revendications 8 à 10 ou cellule bactérienne hôte selon la revendication 11, pour étudier la *trans*-traduction ou l'activité protéasique en lien avec la *trans-*traduction.

13. Procédé de criblage de composés capables d'inhiber la *trans*-traduction bactérienne, comprenant des étapes consistant à:
- incuber une cellule bactérienne transformée par un vecteur ou plasmide selon la revendication 9 ou la revendication 10, avec un composé test, et
- détecter la présence ou l'absence, ou déterminer la quantité, de la protéine codée par le premier gène rapporteur et la détecter la présence ou l'absence, ou déterminer la quantité, de la protéine codée par le deuxième gène rapporteur.

14. Procédé de criblage selon la revendication 13, **caractérisé en ce que** le premier gène rapporteur code pour une première protéine fluorescente et le deuxième gène rapporteur code pour une deuxième protéine fluroescente, et **en ce que** l'étape de détection comprend mesurer la fluorescence émise par première protéine fluorescente et mesurer la fluorescence émise par la deuxième protéine fluroescente.

15. Kit comprenant une construction génétique selon l'une quelconque des revendications 1 à 7 ou un vecteur ou plasmide selon l'une quelconque des revendications 8 à 10 ou une cellule bactérienne hôte selon la revendication 11.


SEQUENCE LISTING

<110>  Université de Rennes 1
       GIUDICE, Emmanuel et al.

<120>  Outil Génétique pour l'Etude de l'Activité trans-Traductionnelle
       et son Utilisation dans le Développement de Nouveaux
       Antibiotiques

<130>  BEP160666EP

<160>  26

<170>  PatentIn version 3.5

<210>  1
<211>  10
<212>  PRT
<213>  Artificial Sequence

<220>
<223>  Etiquette (voir tableau 1)

<400>  1

Ala Asn Asp Glu Asn Tyr Ala Leu Ala Ala
1               5                   10


<210>  2
<211>  39
<212>  DNA
<213>  Artificial Sequence

<220>
<223>  Séquence codant pour l'étiquette de SEQ ID NO: 1

<400>  2
gggcccgcag caaacgacga aaactacgct ctcgcggct                              39


<210>  3
<211>  9
<212>  PRT
<213>  Artificial Sequence

<220>
<223>  Etiquette (voir tableau 1)

<400>  3

Ala Asp Asp Asn Phe Val Leu Ala Ala
1               5


<210>  4
<211>  9
<212>  PRT
<213>  Artificial Sequence

<220>
<223>  Etiquette (voir Tableau 1)

<400>  4

Ala Asp Ser His Gln Arg Leu Ala Ala
1               5


<210>  5
<211>  12
<212>  PRT
<213>  Artificial Sequence

<220>
<223>  Etiquette (voir Tableau 1)

<400>  5

Ala Asp Ser His Gln Arg Asp Tyr Ala Leu Ala Ala
1               5                   10


<210>  6
<211>  12
<212>  PRT
<213>  Artificial Sequence

<220>
<223>  Etiquette (voir tableau 1)

<400>  6

Ala Asp Ser Tyr Gln Arg Asp Tyr Ala Leu Ala Ala
1               5                   10


<210>  7
<211>  12
<212>  PRT
<213>  Artificial Sequence

<220>
<223>  Etiquette (voir tableau 1)

<400>  7

Ala Glu Asn Thr Gln Arg Asp Tyr Ala Leu Ala Ala
1               5                   10


<210>  8
<211>  22
<212>  PRT
<213>  Artificial Sequence

<220>
<223>  Etiquette (voir tableau 1)

<400>  8

Ala Glu Pro Lys Ala Glu Cys Glu Ile Ile Ser Phe Ala Asp Leu Glu
1               5                   10                  15


Asp Leu Arg Val Ala Ala

            20

<210>  9
<211>  15
<212>  PRT
<213>  Artificial Sequence

<220>
<223>  Etiquette (voir tableau 1)

<400>  9

Ala Lys Asn Asn Asn Phe Thr Ser Ser Asn Leu Val Met Ala Ala
1               5                   10                  15


<210>  10
<211>  11
<212>  PRT
<213>  Artificial Sequence

<220>
<223>  Etiquette (voir tableau 1)

<400>  10

Ala Lys Asn Asn Thr Ser Tyr Ala Leu Ala Ala
1               5                   10


<210>  11
<211>  11
<212>  PRT
<213>  Artificial Sequence

<220>
<223>  Etiquette (voir tableau 1)

<400>  11

Ala Lys Asn Thr Asn Ser Tyr Ala Leu Ala Ala
1               5                   10


<210>  12
<211>  10
<212>  PRT
<213>  Artificial Sequence

<220>
<223>  Etiquette (voir tableau 1)

<400>  12

Ala Asn Asp Asp Asn Tyr Ala Leu Ala Ala
1               5                   10


<210>  13
<211>  10
<212>  PRT
<213>  Artificial Sequence

<220>
<223>  Etiquette (voir tableau 1)

<400>  13

Ala Asn Asp Glu Gln Tyr Ala Leu Ala Ala
1               5                   10


<210>  14
<211>  10
<212>  PRT
<213>  Artificial Sequence

<220>
<223>  Etiquette (voir tableau 1)

<400>  14

Ala Asn Asp Glu Arg Phe Ala Leu Ala Ala
1               5                   10


<210>  15
<211>  10
<212>  PRT
<213>  Artificial Sequence

<220>
<223>  Etiquette (voir tableau 1)

<400>  15

Ala Asn Asp Glu Thr Tyr Ala Leu Ala Ala
1               5                   10


<210>  16
<211>  9
<212>  PRT
<213>  Artificial Sequence

<220>
<223>  Etiquette (voir tableau 1)

<400>  16

Ala Asn Asp Asn Phe Ala Leu Ala Ala
1               5


<210>  17
<211>  12
<212>  PRT
<213>  Artificial Sequence

<220>
<223>  Etiquette (voir tableau 1)

<400>  17

Ala Asn Ser Asp Ser Phe Asp Tyr Ala Leu Ala Ala

1               5                   10


<210>  18
<211>  11
<212>  PRT
<213>  Artificial Sequence

<220>
<223>  Etiquette (voir tableau 1)

<400>  18

Gly Lys Ser Asn Asn Asn Phe Ala Val Ala Ala
1               5                   10


<210>  19
<211>  14
<212>  PRT
<213>  Artificial Sequence

<220>
<223>  Etiquette (voir tableau 1)

<400>  19

Val Asn Asn Thr Asp Tyr Ala Pro Ala Tyr Ala Lys Ala Ala
1               5                   10


<210>  20
<211>  56
<212>  DNA
<213>  Artificial Sequence

<220>
<223>  Terminateur synthétique L3S2P56

<400>  20
ctcggaccaa attttcgaaa aaagacgctg aaaagcgtct tttttcgttt tggtcc          56


<210>  21
<211>  90
<212>  DNA
<213>  Artificial Sequence

<220>
<223>  Terminateur naturel ECK12002960

<400>  21
ttcagccaaa aaacttaaga ccgccggtct tgtccactac cttgcagtaa tgcggtggac      60

aggatcggcg gttttctttt ctcttctcaa                                        90


<210>  22
<211>  80
<212>  DNA
<213>  Artificial Sequence

<220>

<223>  terminateur naturel de l'opéron rrnC de E.coli

<400>  22
ccaggcatca aataaaacga aaggctcagt cgaaagactg ggcctttcgt tttatctgtt      60

gtttgtcggt gaacgctctc                                                  80


<210>  23
<211>  226
<212>  DNA
<213>  Artificial Sequence

<220>
<223>  Combinaison des terminateurs de SEQ ID NOs: 20-22

<400>  23
ctcggaccaa attttcgaaa aaagacgctg aaaagcgtct tttttcgttt tggtccttca      60

gccaaaaaac ttaagaccgc cggtcttgtc cactaccttg cagtaatgcg gtggacagga     120

tcggcggttt tcttttctct tctcaaccag gcatcaaata aaacgaaagg ctcagtcgaa     180

agactgggcc tttcgtttta tctgttgttt gtcggtgaac gctctc                    226


<210>  24
<211>  17
<212>  DNA
<213>  Artificial Sequence

<220>
<223>  Première séquence amplificatrice

<400>  24
gtaaaacgac ggccagt                                                     17


<210>  25
<211>  17
<212>  DNA
<213>  Artificial Sequence

<220>
<223>  Deuxième séquence amplificatrice

<400>  25
gtcgtgactg ggaaaac                                                     17


<210>  26
<211>  1899
<212>  DNA
<213>  Artificial Sequence

<220>
<223>  Construction génétique développée par les inventeurs

<400>  26
gtaaaacgac ggccagttaa cttatgcatc tcgagggatc catttaaatt gttgacaatt      60

aatcatcggc tcgtataatg tgtggaattg tgagcggata acaatttgct agcggtaccg     120

aaggagatat acatatggtg agcaagggcg aggaggataa catggccatc atcaaggagt 180

tcatgcgctt caaggtgcac atggagggct ccgtgaacgg ccacgagttc gagatcgagg 240

gcgagggcga gggccgcccc tacgagggca cccagaccgc caagctgaag gtgaccaagg 300

gtggccccct gcccttcgcc tgggacatcc tgtcccctca gttcatgtac ggctccaagg 360

cctacgtgaa gcaccccgcc gacatccccg actacttgaa gctgtccttc cccgagggct 420

tcaagtggga gcgcgtgatg aacttcgagg acggcggcgt ggtgaccgtg acccaggact 480

cctccctgca ggacggcgag ttcatctaca aggtgaagct gcgcggcacc aacttcccct 540

ccgacggccc cgtaatgcag aagaagacca tgggctggga ggcctcctcc gagcggatgt 600

accccgagga cggcgccctg aagggcgaga tcaagcagag gctgaagctg aaggacggcg 660

gccactacga cgctgaggtc aagaccacct acaaggccaa gaagcccgtg cagctgcccg 720

gcgcctacaa cgtcaacatc aagttggaca tcacctccca caacgaggac tacaccatcg 780

tggaacagta cgaacgcgcc gagggccgcc actccaccgg cggcatggac gagctgtaca 840

aggggcccgc agcaaacgac gaaaactacg ctctcgcggc ttaatctaga tttaagaagg 900

agatatacat atgagtaaag gagaagaact tttcactgga gttgtcccaa ttcttgttga 960

attagatggt gatgttaatg ggcacaaatt ttctgtcagt ggagagggtg aaggtgatgc 1020

aacatacgga aaacttaccc ttaaatttat ttgcactact ggaaaactac ctgttccatg 1080

gccaacactt gtcactactt tcgggtatgg tgttcaatgc tttgcgagat acccagatca 1140

tatgaaacag catgactttt tcaagagtgc catgcccgaa ggttatgtac aggaaagaac 1200

tatatttttc aaagatgacg ggaactacaa gacacgtgct gaagtcaagt ttgaaggtga 1260

tacccttgtt aatagaatcg agttaaaagg tattgatttt aaagaagatg gaaacattct 1320

tggacacaaa ttggaataca actataactc acacaatgta tacatcatgg cagacaaaca 1380

aaagaatgga atcaaagtta acttcaaaat tagacacaac attgaagatg gaagcgttca 1440

actagcagac cattatcaac aaaatactcc aattggcgat ggccctgtcc ttttaccaga 1500

caaccattac ctgtccacac aatctgccct ttcgaaagat cccaacgaaa agagagacca 1560

catggtcctt cttgagtttg taacagctgc tgggattaca catggcatgg atgaactata 1620

cagatctctc ggaccaaatt ttcgaaaaaa gacgctgaaa agcgtctttt ttcgttttgg 1680

tccttcagcc aaaaaactta agaccgccgg tcttgtccac taccttgcag taatgcggtg 1740

gacaggatcg gcggttttct tttctcttct caaccaggca tcaaataaaa cgaaaggctc 1800

agtcgaaaga ctgggccttt cgttttatct gttgtttgtc ggtgaacgct gtcgactagt 1860

gagctcaagc ttgaattccg gagtcgtgac tgggaaaac 1899